(19) Europäisches Patentamt
European Patent Office
Office européen des brevets

(11) **EP 1 924 244 B1**

(12) **EUROPÄISCHE PATENTSCHRIFT**

(45) Veröffentlichungstag und Bekanntmachung des Hinweises auf die Patenterteilung:
**27.10.2010  Patentblatt 2010/43**

(21) Anmeldenummer: **06791666.8**

(22) Anmeldetag: **25.08.2006**

(51) Int Cl.:
***A61K 9/00*** (2006.01)

(86) Internationale Anmeldenummer:
**PCT/EP2006/008357**

(87) Internationale Veröffentlichungsnummer:
**WO 2007/028512 (15.03.2007 Gazette 2007/11)**

(54) **MAGNETFELD-GESTEUERTER WIRKSTOFFTRANSFER FÜR DIE AEROSOLTHERAPIE**

MAGNETIC-FIELD CONTROLLED ACTIVE SUBSTANCE TRANSFER FOR AEROSOL THERAPY

TRANSFERT DE PRINCIPES ACTIFS COMMANDE PAR CHAMP MAGNETIQUE DANS LE CADRE D'UNE AEROSOLTHERAPIE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **08.09.2005  DE 102005042768**

(43) Veröffentlichungstag der Anmeldung:
**28.05.2008  Patentblatt 2008/22**

(73) Patentinhaber: **Ludwig-Maximilians-Universität 80539 München (DE)**

(72) Erfinder:
• **Rudolph, Carsten**
  **80801 München (DE)**
• **Rosenecker, Joseph**
  **82541 Münsing (DE)**

(74) Vertreter: **Graf von Stosch, Andreas et al Graf von Stosch Patentanwaltsgesellschaft mbH Prinzregentenstrasse 22 80538 München (DE)**

(56) Entgegenhaltungen:
**WO-A-2004/108165     WO-A-2004/112799**

• **ALLY J ET AL: "Magnetic targeting of aerosol particles for cancer therapy" JOURNAL OF MAGNETISM AND MAGNETIC MATERIALS, ELSEVIER SCIENCE PUBLISHERS, AMSTERDAM, NL, Bd. 293, Nr. 1, Mai 2005 (2005-05), Seiten 442-449, XP004883016 ISSN: 0304-8853 in der Anmeldung erwähnt**
• **YU. I. DIKANSKII; V. V. KISELEV: "Magnetosensitive aerosols and prospects of their application" MAGNETOHYDRODYNAMICS, Bd. 34, Nr. 3, 1998, Seiten 212-215, XP009080735**

EP 1 924 244 B1

**Beschreibung**

Die vorliegende Erfindung betrifft Magnetpartikel-haltige Aerosole, wobei die Aerosole Magnetpartikel sowie einen pharmazeutischen Wirkstoff umfassen. Die Erfindung betrifft ferner die Verwendung solcher Magnetpartikel-haltigen Aerosole zum gerichteten Magnetfeld-gesteuerten Transfer der enthaltenen Wirkstoffe in der Aerosoltherapie.

Aeorosole sind feste oder flüssige schwebende Partikel in Gasen (insbesondere Luft) mit einem Durchmesser von etwa 0,0001 μm bis etwa 100 μm, wobei die Zusammensetzung und Form der Aerosole sehr stark variieren kann. Liegen im Aerosol feste Partikel vor, werden solche Aerosole typischerweise als Rauche oder Staube bezeichnet, liegen im Aerosol dagegen flüssige Partikel vor, werden diese Aerosole typischerweise als Nebel bezeichnet. Daneben können auch Mischformen dieser Aerosole auftreten, d.h. Aerosole, mit festen und flüssigen schwebenden Partikeln. In den letzten Jahrzehnten wurden zahlreiche künstliche Aerosole für einen weiten industriellen und wirtschaftlichen Anwendungsbereich hergestellt. Derartige künstliche Aerosole lassen sich durch übliche Dispersions- und Kondensationsverfahren herstellen und werden in der Regel in Verbindung mit einem verflüssigten Druckgas als Treibgas in Sprühdosen verwendet. Sie finden, je nach Art der enthaltenen Partikel, bspw. Anwendung als Haar- und Körperpflegemittel, Desodorantien, Parfums, Geruchsverbesserer, Desinfektions- und Schädlingsbekämpfungsmittel, Fußboden-, Glas- und Möbelpflegemittel, Lacke und Anstrichmittel, Autopflegemittel etc.. Besondere Anwendung finden Aerosole mit und ohne Treibgas auch auf dem Gebiet der Medizin, in der sog. Aerosoltherapie zur Behandlung diverser Atemwegs- und/oder Lungenerkrankungen. Durch die letztgenannten medizinischen Aerosole können pharmazeutische Wirkstoffe, z.B. Salbutamol, Formoterol, lpatropiumbromid, Budesonid, Fenoterol, Terbutalin, Tiotropiumbromid, Salmeterol, Beclometason, Fluticason, Mometason, Tobramycin, Theophyllin, Dornase α, α1-Antitrypsin, Interferon-β, Insulin, Calcitonin oder Wachstumshormone, über die Lunge verabreicht werden. Die vorliegende Erfindung und nachfolgende Beschreibung bezieht sich auf solche medizinischen Aerosole.

Die kleinsten (pharmazeutisch wirksamen) Partikel in medizinischen Aerosolen sind z.B. Nukleinsäuren, Peptide oder Proteine, während die größten Partikel z.B. Nebelpartikel sind. Häufig bestehen die Aerosole aus Gemischen von Partikeln unterschiedlicher Partikelgrößen und verkörpern somit eine polydisperse Größenverteilung. Allgemein ist es bei der Betrachtung der Größenverteilung von Aerosolen von Bedeutung, ob sich die Betrachtung auf die Anzahl, die Oberfläche oder die Masse der Partikel bezieht (wobei in diesem Zusammenhang zu beachten ist, dass bspw. ein Partikel des Durchmessers von 10 μm der Masse von 1000 Partikel des Durchmessers von 1 μm entspricht). Das Spektrum der Größenverteilung wird allgemein durch eine Kennzahl, dem medianen aerodynamischen Massendurchmesser (Mass Median Aerodynamic Diameter; MMAD), festgelegt, wobei in der Regel 50 % der Aerosolmasse größer und 50 % kleiner als der MMAD ist. In diesem Zusammenhang ist anzumerken, dass insbesondere für biologische Systeme in der Regel die aerodynamische Massenverteilung des Aerosolspektrums herangezogen wird, wie sie bspw. von Köhler, D. & Fischer, F. in Theorie und Praxis der Inhalationstherapie (Arcis Verlag GmbH, München, 2000) beschrieben wird.

Medizinische Aerosole werden in der Aerosoltherapie typischerweise von dem zu behandelnden Patienten oral oder nasal inhaliert. Während oder nach der Inhalation der Partikel in die Lunge weicht ein gewisser Anteil der Partikel von der durch das Ein- oder Ausatmen gebildeten Stromlinie des Aerosols ab und gelangt dabei in Kontakt mit der feuchten Oberfläche der Lufträume, z.B. dem, Hals-, Nasen- oder Rachenraum, der Luftröhre oder dem Lungengewebe. Dieses Phänomen wird im allgemeinen als Partikeldeposition oder Deposition bezeichnet und unterliegt insbesondere den folgenden drei physikalischen Mechanismen:

-   Impaktion,
-   Sedimentation und
-   Diffusion.

Bei der Impaktion folgen die Aerosolpartikel bis zu einem bestimmten Durchmesser der Stromlinie des inhalierten Aerosols. Ab einem Durchmesser von 2 bis 3 μm wird die Massenträgheit des Aerosols relevant, was bedeutet, dass die Aerosolpartikel die Tendenz aufweisen, bei Richtungsänderungen der Stromlinie des Aerosols geradeaus zu fliegen, und an der Oberfläche zu deponieren. Solche Richtungsänderungen der Stromlinie des Aerosols erfolgen insbesondere aufgrund der physiologischen Form der Atemwege, z.B. dem Oropharynx, den Verzweigungen der Atemwege zum linken bzw. rechten Lungenflügel und/oder den Verzweigungen im Bereich der Alveolen. Die Depositionswahrscheinlichkeit (DE) ist bei der Impaktion proportional dem Quadrat des Durchmessers (d) und dem Aerosolfluss (V):

$$DE \sim d^2 \cdot V$$

Die Impaktion stellt einen wesentlichen Depositionsmechanismus für Aerosole in dem Größenbereich <u>oberhalb</u>

eines Partikeldurchmessers von 3 μm dar. Die Abscheidung durch Impaktion ist daher überall dort zu beobachten, wo hohe Aerosolgeschwindigkeiten und starke Richtungsänderungen auftreten, wie dies größtenteils in den großen Atemwegen und dem Oropharynx der Fall ist. Vor allem größere Partikel oberhalb von 10 μm werden an der ersten starken Richtungsänderung, d.h. dem Oropharynx zu über 90 % abgeschieden (siehe z.B. Köhler, D. & Fischer (2000, supra); Schulz, H. & Muhle, H. 323-345 (Academic Press, 2000)).

Bei der Sedimentation handelt es sich um einen Depositionsmechanismus, der auf die Schwerkraft der Aerosolpartikel zurückzuführen ist. Auch hier hängt die Deposition von der Partikelgröße ab und erfolgt insbesondere bei einem Partikeldurchmesser oberhalb von 0,5 bis 1 μm. Die dabei bestimmende Sinkgeschwindigkeit ($v_s$) lässt sich annähernd durch die folgende Gleichung beschreiben:

$$V_s = \frac{d^2 \cdot g \cdot \rho}{18 \cdot \eta}$$

Die Sinkgeschwindigkeit ($v_s$) hängt ab von dem Quadrat des Partikeldurchmessers (d), der Gravitationskonstante (g), der Partikeldichte (ρ) und der Viskosität (η) des Gases (siehe z.B. Köhler, D. & Fischer (2000, supra)).

Im Unterschied zu Impaktion und Sedimentation folgen bei der Diffusion dagegen Partikel mit einem Durchmesser von kleiner als 1 μm einerseits noch dem Gastrom, d.h. der Stromlinie, andererseits ähneln sie immer stärker Molekülen, die der Brownschen Molekularbewegung unterworfen sind. Aufgrund der Diffusion können diese Partikel daher in der Wandnähe ihre anfängliche Stromlinie verlassen und an der Wand deponieren. Die mittlere Fortbewegungsstrecke eines Partikels (Δ) bei der Diffusion berechnet sich folgendermaßen:

$$\Delta = \sqrt{\frac{2 \cdot k \cdot T \cdot C \cdot t}{\pi \cdot \eta \cdot d}}$$

Die mittlere Fortbewegungsstrecke eines Partikels (Δ) bei der Diffusion hängt damit von dem Partikeldurchmesser (d), der Viskosität des Gases (η), der Zeit (t) und der Temperatur (T in °K) ab. Die Konstanten der Formel sind: C = Cunninghamsche Gleitkorrektur und k = Boltzmann Konstante. Die Diffusionsstrecke ähnelt etwa einer glockenförmigen Häufigkeitsverteilung um den Ausgangswert, wobei die mittlere Fortbewegungsstrecke proportional zur Wurzel der Variablen ist (siehe z.B. Köhler, D. & Fischer (2000, supra)).

In Tabelle 1 sind zur Übersicht die zurückgelegten Wegstrecken mit Hinblick auf Sedimentation und Diffusion vergleichend dargestellt. Es ist zu erkennen, dass kleine Partikel erhebliche Strecken über Diffusion zurücklegen können.

Tabelle 1: Sedimentations- und Diffusionsstrecke bei 6 verschiedenen Partikeldurchmessern (siehe z.B. Köhler, D. & Fischer (2000, supra)).

| Partikeldurchmesser [μm] | Sedimentation [μm/s] | Diffusion [μm/s] |
|---|---|---|
| 0,01 | 0,07 | 340 |
| 0,1 | 0,7 | 38 |
| 1 | 38 | 8 |
| 5 | 740 | 3 |
| 10 | 2.910 | 2 |
| 100 | 72.000 | 1 |

Für die therapeutische Anwendung von Aerosolen ist der Depositionsmechanismus von entscheidender Bedeutung für die Wahl der Partikelgröße verschiedener Wirkstoffe zur Therapie und/oder Prophylaxe diverser Atemwegs- und/oder Lungenerkrankungen. Daneben ist jedoch auch insbesondere die Frage von erheblicher Bedeutung, in welchen Regionen die inhalierten Partikel in der Lunge deponieren sollen. Detaillierte Untersuchungen im Stand der Technik haben ergeben, dass unter bestimmten Umständen (z.B. der oben beschriebenen Partikelgröße oder der Atemtechnik, d.h. der Art und Weise des Atmens) die Deposition in bestimmten Kompartimenten des Bronchialbaums bzw. Alveolargebiets bevorzugt erfolgt. Für eine effektive und schonende Therapie ist es wünschenswert, die Aerosole und die durch diese Aerosole transportierten pharmazeutischen Wirkstoffe zielgerichtet nur definierten, erkrankten Regionen der Lunge zu verabreichen. Zum einen kann hierdurch die Dosis des zu verabreichenden pharmazeutischen Wirkstoffs vermindert werden, zum anderen können unerwünschte Nebenwirkungen auf das umgebende gesunde Gewebe reduziert bzw. vermieden werden.

Um dieses Ziel zu erreichen, ergibt sich das Problem, dass die natürliche oder "normale" Verbreitung der inhalierten Aerosole (z.B. im Bronchialbaum bzw. Alveolargebiet) beeinflusst werden müsste, wenn eine Verabreichung an andere Regionen gewünscht ist, um eine gerichtete Verbreitung in solche definierten Regionen der Lunge erzielen zu können. In diesem Zusammenhang wurden im Stand der Technik verschiedene Mechanismen zur gerichteten Aerosolzufuhr in die Lunge vorgeschlagen, wie bspw. von Ernst, N. et al. (Interaction of liposomal and polycationic transfection complexes with pulmonary surfactant. J Gene Med 1, 331-40. (1999)) und Rosenecker, J. et al. (Interaction of bronchoalveolar lavage fluid with polyplexes and lipoplexes: analysing the role of proteins and glycoproteins. J Gene Med 5, 49-60. (2003)) beschrieben. So wurden bspw. Veränderungen der Partikelgröße in Verbindung mit verschiedenen Atmungstechniken, z.B. langes Anhalten des Atems oder auch eine extrem langsame Inhalation von Aerosolen vorgeschlagen. Ebenfalls wurde die individuelle Atemwegsgeometrie der Patienten berücksichtigt. Weiterhin wurde für bestimmte Wirkstoffe, z. B. für einen DNA-Transfer im Rahmen der Gentherapie, die Verwendung von viralen oder liposomalen Vektoren in Epithelzellen der Luftwege zur Behandlung von Mukoviszidose beschrieben (Rudolph, C. et al. Nonviral gene delivery to the lung with copolymer-protected and transferrin-modified polyethylenimine. Biochim Biophys Acta 1573, 75-83. (2002)).

Ally et al. (Journal of Magnetism and Magnetic Materials 293, 442-449 (2005)), beschreiben hypothetisch die Möglichkeit eines Magnet-gesteuerten Aerosolpartikeltransports. Hierzu wird vorgeschlagen, chemotherapeutische Wirkstoffe mit Hilfe eines magnetischen Feldes in Regionen der Lunge, die von Lungenkrebs befallen sind, zu steuern. Den Untersuchungen liegt jedoch lediglich ein *in vitro* Modell zugrunde, in dem feste Carbonyleisen-Partikel mit einem Durchmesser von 1 bis 3 $\mu$m in Luft verwendet werden. Die hier verwendete Partikelgeschwindikeit von v = 0,34 m/s in Verbindung mit der Magnetfeldstärke von 36 mT für die angegebene Partikelgröße von 1 bis 3 $\mu$m ist allerdings auf die beschriebenen *in vitro* Untersuchungen abgestimmt und nicht auf die abweichenden *in vivo* Bedingungen übertragbar. Untersuchungen der vorliegenden Erfindung haben ergeben, dass unter den von Ally *et al.* (2005, supra) gewählten Bedingungen *in vivo* nur ein sehr unzureichender Partikeltransport erfolgt. *In vivo* Bedingungen, die in einem *in vitro* System nicht auftreten, wie die Behinderung des Aerosoltransports durch physiologische Bedingungen, Enzyme, Schleimhäute, Aufbau und Konstruktion der Atemwege etc., finden im übrigen bei Ally *et al.* (2005, supra) keine Berücksichtigung. Mit anderen Worten kann ein effektiver Partikeltransport *in vivo,* wie er in der Aerosoltherapie benötigt wird, mit den in Ally *et al.* (2005, supra) angegebenen Parametern nicht erzielt werden.

Zusammenfassend bleibt festzustellen, dass keine der im Stand der Technik beschriebenen Vorgehensweisen dazu geführt hat, einen gezielten Transport und eine gerichtete Deponierung von Aerosolpartikeln und damit von pharmazeutischen Wirkstoffen in definierte Regionen der Lunge zu gewährleisten. Dies hat den Nachteil, dass erhöhte Mengen pharmazeutischer Wirkstoffe verabreicht werden müssen, um die beabsichtigte Wirkung in dem zu behandelnden erkrankten Gewebe der Lunge zu erzielen, was in der Folge zu erhöhten Wirkstoffkosten und folglich auch zu erhöhten Therapiekosten führt. Ein weiterer gravierender Nachteil besteht darin, dass die Wirkstoff-beladenen Aerosole nicht nur in erkrankten Regionen der Atemwege oder Lunge deponiert werden, sondern auch in Regionen, die nicht von der Erkrankung betroffen sind, d.h. in gesundem Gewebe, deponiert werden. Dieses Depositionsmuster ist nachteilig, da unerwünschte Nebenwirkungen auf nicht erkranktes Atemwegs- bzw. Lungengewebe durch den Kontakt bzw. die Aufnahme des verabreichten Wirkstoffs auftreten können. Diese Überlegungen verdeutlichen, dass neue Methoden erforderlich sind, welche eine gerichtete lokale Deposition von Aerosolen in den Atemwegen und insbesondere in der Lunge ermöglichen.

Aufgabe der vorliegenden Erfindung ist es, ein System bereitzustellen, durch das ein gerichteter *in vivo* Transport von Aerosolen in definierte Regionen der Atemwege und der Lunge gewährleistet wird.

Diese Aufgabe wird durch den Gegenstand des Anspruchs 1 der vorliegenden Erfindung gelöst. Vorteilhafte Ausführungsformen der Erfindung werden in den weiteren Ansprüchen beschrieben.

Die vorliegende Erfindung betrifft in einem ersten Gegenstand ein Magnetpartikel-haltiges Aerosol, wobei das Aerosol Magnetpartikel mit einem Durchmesser von mindestens 5 nm und höchstens 800 nm sowie mindestens einen pharmazeutischen Wirkstoff enthält. Bevorzugt weisen die enthaltenen Magnetpartikel einen Durchmesser von mindestens 50 nm und höchstens 750 nm, weiter bevorzugt von mindestens 100 nm und höchstens 700 nm, stärker bevorzugt von mindestens 150 nm und höchstens 600 nm, noch stärker bevorzugt von mindestens 200 nm und höchstens 500 nm, insbesondere bevorzugt von mindestens 250 nm und höchstens 450, am stärksten bevorzugt von mindestens 300 nm und höchstens 400 nm auf.

Der Erfindung liegen Untersuchungen zugrunde, mit denen erstmals nachgewiesen werden konnte, dass ein inhaliertes erfindungsgemäßes Magnetpartikel-haltiges Aerosol, das einen pharmazeutischen Wirkstoff enthält, gerichtet, d.h. gezielt, in definierte Bereiche der Lunge transportiert werden kann. Der Transport dieses erfindungsgemäßen Magnetpartikel-haltigen Aerosols in definierte Regionen der Lunge erfolgt über ein extern angelegtes magnetisches Feld, das eine Deponierung der Magnetpartikel und folglich auch des Aerosols auf der Oberfläche des gewünschten Lungenbereichs bewirkt. Die vorliegende Erfindung stellt damit ein effektives, *in vivo* anwendbares, Aerosol für den gerichteten Wirkstofftransport in der Aerosoltherapie bereit. Der Begriff "*in vivo*" bedeutet in diesem Zusammenhang jede Anwendung des erfindungsgemäßen Magnetpartikel-haltigen Aerosols am Körper eines lebenden mehrzelligen Lebewesens, bevorzugt eines Säugetiers, stärker bevorzugt eines Menschen. Im Gegensatz dazu bedeutet in diesem Zusammenhang "*in vitro*" jede Anwendung des erfindungsgemäßen Magnetpartikel-haltigen Aerosols außerhalb eines solchen Körpers bzw. Lebewesens.

Die in dem erfindungsgemäßen Magnetpartikel-haltigen Aerosol enthaltenen Magnetpartikel können aus verschiedenen Metallen und deren Oxiden oder Hydroxiden bestehen oder diese enthalten. Erfindungsgemäß geeignete Magnetpartikel sind bspw. in der Internationalen Patentanmeldung WO 02/000870 beschrieben, deren diesbezüglicher Offenbarungsgehalt Gegenstand der vorliegenden Erfindung ist. Der Begriff "Magnetpartikel" bedeutet magnetisch reagierende Festphasen. Diese Festphasen sind typischerweise Partikel oder Aggregate hiervon mit einem Durchmesser im Nano- bis Mikrometerbereich, nicht größer als 800 nm, und enthalten üblicherweise ein oder mehrere Metalle oder Oxide oder Hydroxide davon, die auf die Magnetkraft eines magnetischen Feldes reagieren und vorzugsweise von der Quelle des magnetischen Feldes angezogen oder in eine definierte Richtung beschleunigt werden. Ebenfalls umfasst sind temporär magnetische Partikel, bspw. aus ferrimagnetischen oder bevorzugt ferromagnetischen Materialien. Weiterhin sind Partikel aus paramagnetischem oder superparamagnetischem Material von der vorliegenden Erfindung umfasst. Geeignete Materialien der Magnetpartikel umfassen bspw. Eisen, Kobalt oder Nickel, magnetische Eisenoxide oder -hydroxide, wie $Fe_3O_4$, gamma-$Fe_2O_3$, oder Doppel-Oxide oder -Hydroxide aus zwei- oder dreiwertigen Eisenionen mit zwei- oder dreiwertigen anderen Metallionen, z.B. $Co^{2+}$, $Mn^{2+}$, $Cu^{2+}$, $Ni^{2+}$, $Cr^{3+}$, $Gd^{3+}$, $Dy^{3+}$ oder $Sm^{3+}$, sowie beliebige Mischungen solcher Oxide oder Hydroxide. Herstellungsverfahren von Magnetpartikeln werden z.B. in Schwertmann U. und Cornell R. M., Iron Oxides in the Laboratory, VCH Weinheim 1991, in WO 02/000870 und in DE 196 24 426 beschrieben.

Die in dem erfindungsgemäßen Magnetpartikel-haltigen Aerosol enthaltenen Magnetpartikel sind typischerweise künstliche Magnetpartikel, d.h. nicht von einer biologischen Quelle (einem lebenden Lebewesen) erhältlich. Vorzugsweise induzieren die Magnetpartikel oder deren Aggregate keine systemischen toxischen Nebenwirkungen in dem Lebewesen, dem sie verabreicht werden. Gemäß einer Ausführungsform der Erfindung sind die in dem erfindungsgemäßen Magnetpartikel-haltigen Aerosol enthaltenen Magnetpartikel an beliebige, hierin beschriebene Vektoren, Liposomen, Hohlkolloide, Nanopartikel oder an den/die pharmazeutischen Wirkstoff(e) selbst gebunden. Die in dem erfindungsgemäßen Magnetpartikel-haltigen Aerosol enthaltenen Magnetpartikel der vorliegenden Erfindung können unbeschichtet oder beschichtet vorliegen. Falls die Magnetpartikel beschichtet vorliegen, wird die Beschichtung bevorzugt ausgewählt aus positiv oder negativ geladenen Elektrolyten, wie Phosphaten, Citraten oder Aminen, mit Silanen, Fettsäuren oder Polymeren, z.B. Polysacchariden, Proteinen oder natürlichen oder synthetischen Polymeren. Eine solche Beschichtung der in dem erfindungsgemäßen Magnetpartikel-haltigen Aerosol enthaltenen Magnetpartikel dient bspw. zur Reduktion einer eventuellen Toxizität der Magnetpartikel, zu der Ankopplung von dem/den pharmazeutischen Wirkstoff(en) an die. Magnetpartikel, zur Verbesserung/Erhöhung des Membrandurchtritts (des Wirkstoffs, ggf. zusammen mit dem/den Magnetpartikel(n)), usw.. Beispiele solcher Beschichtungen sind u.a. in US 4,554,088, US 4,554, 089, US 4,208,294, US 4,101,435 und DE 196 24 426 beschrieben, deren diesbezüglicher Offenbarungsgehalt von der vorliegenden Erfindung vollständig umfasst ist. Diese Beschichtungen, bzw. die dazu verwendeten Verbindungen, können, wie im folgenden beschriebene, reaktive funktionale Gruppen aufweisen. Diese reaktiven funktionalen Gruppen können jedoch auch bei Bedarf nach dem Beschichtungsvorgang durch übliche chemische Modifikationen eingefügt werden. Solche funktionalen Gruppen können Kationenaustauscheigenschaften besitzen, wie bspw. Xanthat-, Xanthid-, Dicarboxyl-, Carboxymethyl-, Sulfonat-, Sulfat-, Triacetat-, Phosphonat-, Phosphat-, Citrat-, Tartrat-, Carboxylat- öder Laktatgruppen von natürlichen oder synthetischen Polymeren, wie Polysacchariden, Polyvinylalkohol (PVA), Polyvinylpyrrolidon (PVP) oder Polyethylenglykol (PGP). Diese funktionalen Gruppen können z.B. vor oder nach der Beschichtung der Magnetpartikel in die oben beschriebenen natürlichen oder synthetischen Polymere eingebaut werden.

Wie bereits zuvor beschrieben, erfolgt der gerichtete Transport des erfindungsgemäßen Magnetpartikel-haltigen Aerosols, d.h. der Magnetpartikel- und Wirkstoff-Transport, über ein extern angelegtes magnetisches Feld, mit dem die inhalierten Magnetpartikel und der/die pharmazeutische(n) Wirkstoff(e) in die definierten Regionen der Atemwege, bevorzugt der Lunge, gesteuert werden. Ein im Sinne der Erfindung geeignetes "magnetisches Feld" bezieht sich auf ein durch einen Magneten als Quelle erzeugtes magnetisches Feld, das der Form und Feldstärke nach geeignet ist, die erfindungsgemäßen Magnetpartikel nebst Wirkstoff(en) entgegen anderer auf diese wirkenden physikalischen Phänomene anzuziehen. Solche anderen Phänomene können bspw. oben beschriebene Diffusions-, Sedimentations- und/ oder Impaktionsvorgänge darstellen. Geeignete magnetische Felder für die erfindungsgemäßen *in vivo* Anwendungen sollten vorzugsweise eine Feldstärke von mindestens 100 mT (Millitesla), bevorzugt von mindestens 200 mT, ebenfalls bevorzugt von mindestens 500 mT, weiterhin bevorzugt von mindestens 1T (Tesla) und mehr erzeugen. Der von den magnetischen Feldern erzeugte Magnetfeldgradient sollte vorzugsweise größer als 1 T/m, stärker, bevorzugt größer als 10 T/m sein. Ein "Magnet" im Sinne der vorliegenden Erfindung, der solche vorbeschriebenen magnetischen Felder erzeugt, kann jeder hierzu geeignete Magnet sein. Einsetzbar im Sinne der vorliegenden Erfindung sind bspw. Permanentmagneten oder (durch elektrischen Strom betriebene) Elektromagneten. Die Steuerung der Intensität (der Feldstärke des Magneten) erfolgt typischerweise über geeignete Mess- bzw. Steuerungsinstrumente, die mit dem Magneten verbunden sind.

Gemäß einer bevorzugten Ausführungsform ist das extern angelegte magnetische Feld permanent vorhanden, d.h. das erfindungsgemäße Magnetpartikel-haltige Aerosol kann nach Verabreichung zu jedem Zeitpunkt aus der durch das Ein- und Ausatmen bedingten Stomlinienbahn abgelenkt und auf der Oberfläche der Atemwege deponiert werden, sofern die darin enthaltenen Magnetpartikel in einen dafür ausreichenden Bereich der magnetischen Feldstärke gelangen. Ein solches permanentes Magnetfeld kann durch jeden der hier beschriebenen Magneten erzeugt werden und weist bevorzugt die allgemeinen Eigenschaften eines wie oben beschriebenen Magnetfeldes auf. Bevorzugt ist das permanente Magnetfeld für mindestens den Zeitraum der Behandlung, d.h. der Verabreichung oder Inhalation des erfindungsgemäßen Magnetpartikel-haltigen Aerosols, aktiv.

Gemäß einer anderen bevorzugten Ausführungsform ist das extern angelegte magnetische Feld nicht permanent vorhanden, bevorzugt nur für einen Teil des Zeitraums der Behandlung, d.h. eines Teils des Zeitraums der Verabreichung oder Inhalation des erfindungsgemäßen Magnetpartikel-haltigen Aerosols, aktiv. Stärker bevorzugt ist das Magnetfeld lediglich während des Zeitraumes der Ruhephasen zwischen Einatmung und Ausatmung oder zwischen Ausatmung und Einatmung aktiv. Eine solche nicht-permanente Aktivierung des Magnetfeldes sorgt bevorzugt für die Deposition der erfindungsgemäßen Magnetpartikel-haltigen Aerosole auf der Oberfläche während dieser Ruhephasen und ermöglicht damit eine gleichmäßige Verteilung des erfindungsgemäßen Magnetpartikel-haltigen Aerosols. Gemäß einer besonders bevorzugten Ausführungsform kann die Steuerung des Magnetfeldes in Koordination mit der Atmung des Patienten dynamisch in Abhängigkeit des Atemrhythmus des Patienten erfolgen, so dass während des Ein- und Ausatmens des Patienten im zu therapierenden Bereich kein Magnetfeld anliegt, jedoch in den Ruhephasen dort ein Magnetfeld anliegt und nur dann eine Deposition des erfindungsgemäßen Magnetpartikel-haltigen Aerosols auf der Oberfläche der Atemwege erfolgt. Bevorzugt ist dazu die Luft über der ausgewählten Oberfläche der Atemwege durch das erfindungsgemäße Magnetpartikel-haltige Aerosol derart gesättigt, daß innerhalb von mehreren Aktivierungen des Magnetfeldes/der Magnetfelder durch den/die Magneten eine signifikante Deposition des transportierten Wirkstoffs bzw. des erfindungsgemäßen Magnetpartikel-haltigen Aerosols auf der ausgewählten Oberfläche der Atemwege ermöglicht wird. Die Steuerung des Magneten kann bspw. durch eine elektrische Schaltung ermöglicht werden, die am Anfang und Ende der Einatmung bzw. Ausatmung des Patienten im Vernebler oder Inhalator ein Signal auslöst, durch das wiederum das Magnetfeld eines wie hier beschriebenen Magneten an- bzw. abgeschaltet wird. Das An- bzw. Abschalten des Magnetfeldes kann bei permanenten Magneten z.B. durch mechanisches Entfernen bzw. Wegdrehen der Pole des Magneten erfolgen, bei Elektro-Magneten ebenfalls mechanisch, bevorzugt jedoch z.B. durch An- bzw. Abschalten des zum Erzeugen des Magnetfeldes erforderlichen elektrischen Stromes.

Gemäß einer weiteren bevorzugten Ausführungsform handelt es sich bei dem extern angelegten magnetischen Feld um ein pulsierendes magnetisches Feld. Ein "pulsierendes magnetisches Feld" bedeutet vorliegend insbesondere, dass die Feldstärke des magnetischen Feldes im zu therapierenden Bereich ab- bzw. zunimmt, üblicherweise periodisch oder nahezu periodisch. Dabei wird bevorzugt im Maximum des Pulses die maximale gewünschte Feldstärke erreicht, während im Minimum des Pulses bevorzugt eine möglichst geringe, stärker bevorzugt eine weniger als 20%ige, noch stärker bevorzugt eine weniger als 10%ige Feldstärke der zuvor angelegten Feldstärke, und noch weiter bevorzugt keine Feldstärke anliegt. Besonders bevorzugt wird das pulsierende magnetische Feld mit der Atmung des Patienten dynamisch in Abhängigkeit des Atemrhythmus des Patienten so koordiniert, dass das Maximum des Pulses in einer Ruhephase zwischen Einatmung und Ausatmung bzw. zwischen Ausatmung und Einatmung liegt, während das Minimum des Pulses während der Einatmung bzw. Ausatmung liegt. Das pulsierende magnetische Feld kann dabei das "Profil" eines Rechteckpulses, eines sinusoidalen Pulses, etc., oder Annäherungen an diese Profile aufweisen. Der Puls kann hier, wie zuvor durch An- bzw. Abschalten des Magnetfeldes, z.B. bei permanenten Magneten durch mechanisches Entfernen bzw. Wegdrehen der Pole des Magneten, bei Elektro-Magneten ebenfalls mechanisch, bevorzugt jedoch z.B. durch An- bzw. Abschalten des zum Erzeugen des Magnetfeldes erforderlichen elektrischen Stromes. Das pulsierende magnetische Feld kann weiterhin, bei Verwendung eines Elektromagneten mit Gleichstrom oder mit Wechselstrom erzeugt werden. Bei Betrieb eines Elektromagneten mit Gleichstrom wird bevorzugt ein magnetisches Feld erzeugt, das seine Richtung (+/- -Polung) nicht verändert. Das entstehende magnetische Feld kann dabei durch einen Fachmann, je nach Bedarf, entsprechend der Polung eingestellt werden (+/- - oder -/+ -Polung)

Gemäß einer weiteren bevorzugten Ausführungsform handelt es sich bei dem extern angelegten magnetischen Feld um ein oszillierendes magnetisches Feld. Unter dem Begriff "oszillierendes magnetisches Feld" im Sinne der vorliegenden Erfindung ist ein magnetisches Feld zu verstehen, das periodisch seine Richtung (+/- -Polung) verändert. Ein solches oszillierendes Magnetfeld wird typischerweise durch Verwendung eines Elektromagneten und Betrieb des Elektromagneten mit Wechselstrom erzeugt. Eine durch ein oszillierendes Magnetfeld bewirkte Richtungsänderung des Magnetfeldes (d.h. Änderung der +/- -Polung) kann bevorzugt eine kinetische Energie auf die Magnetpartikel ausüben, unter deren Einfluss der Transport des erfindungsgemäßen Magnetpartikel-haltigen Aerosols und/oder bspw. die Freisetzung des im erfindungsgemäßen Magnetpartikel-haltigen Aerosol an die Magnetpartikel gebundenen pharmazeutischen Wirkstoffes begünstigt, bewirkt oder beschleunigt wird. Ein oszillierendes Magnetfeld permanent vorliegen, oder, wie zuvor beschrieben, an den Atemrhythmus des Patienten derart angepasst werden, so dass während des Ein- und Ausatmens des Patienten im zu therapierenden Bereich kein oszillierendes Magnetfeld anliegt, jedoch in den Ruhephasen dort ein oszillierendes Magnetfeld anliegt.

Alle zuvor beschriebenen Ausführungsformen können in geeigneter Weise auch miteinander kombiniert werden. So kann bspw. ein pulsierendes magnetisches Feld oszillierend betrieben werden (pulsierendes oszillierendes magnetisches Feld), d.h. das magnetische Feld weist einen charakteristischen Puls auf (z.B. Rechteck- oder sinusoidaler Puls), der bspw. in seinem Maximum oszilliert oder von Maximum zu Minimum einen, stetig in der Feldstärke abnehmenden, oszillierenden Verlauf aufweist, etc..

Der gerichtete Transport des erfindungsgemäßen Magnetpartikel-haltigen Aerosols, d.h. der Magnetpartikel- und Wirkstoff-Transport, erfolgt durch einen extern angeordneten Magneten, d.h. außerhalb des zu behandelnden Lebewesens (Säugetier, bevorzugt Mensch). Der gerichtete Transport in definierte Regionen der Atemwege, z.B. der Lunge, erfolgt nach Inhalation des erfindungsgemäßen Magnetpartikel-haltigen Aerosols durch das zu behandelnde Lebewesen (Säugetier, bevorzugt Mensch) vorzugsweise über entsprechende Positionsänderung(en) des externen Magneten zu den definierten Regionen. Demnach ist es vorteilhaft, wenn der Magnet frei beweglich ist. Frei beweglich bedeutet bspw., dass der Magnet manuell geführt/bewegt wird. Es ist jedoch auch möglich und vorteilhaft, dass der Magnet an einer Vorrichtung, z. B. einem Gestell, bewegbar angebracht ist, an welcher seine Position manuell, elektronisch oder Computer-gesteuert veränderbar, insbesondere schwenkbar, höhenverstellbar und arretierbar ist. Eine weitere Möglichkeit der Positionierung des/der externen Magneten ist in diesem Zusammenhang die Anordnung mehrerer Magneten, z.B. durch eine Anordnung von Permanentmagneten oder Elektromagneten in Reihe(n) oder als Bügel oder als "Sandwich"-Konstruktion, wobei diese Magneten bevorzugt das zu therapierenden Bereich abdecken. Die Magneten können entweder nacheinander oder gleichzeitig aktiviert werden. Durch eine solche Anordnung mehrerer Magneten kann z.B. eine größere Region zur Deponierung des erfindungsgemäßen Magnetpartikel-haltigen Aerosols und damit des zu therapierenden Bereichs ausgewählt werden.

Die für einen effektiven gerichteten Transport erforderliche. Geschwindigkeit der Magnetpartikel und pharmazeutischen Wirkstoffe des erfindungsgemäßen Magnetpartikel-haltigen Aerosols in der Lunge ist von mehreren Faktoren abhängig, bspw. davon, in welche Regionen der Lunge das inhalierte Aerosol zu transportieren ist, von dem Durchmesser der Magnetpartikel, von der Größe der pharmazeutischen Wirkstoffkomponente(n), der individuellen Atemwegsgeometrie des behandelten Lebewesens usw.. Die Geschwindigkeit kann daher bspw. beeinflusst werden durch Magnetpartikeldurchmesser, Wirkstoffgröße, Atmungstechnik, z.B. schnelles oder langsames Einatmen, tiefes oder flaches Atmen, Anhalten des eingeatmeten Atems, sowie der an dem/den externen Magneten angelegen Feldstärke oder ein oszillierend und/oder pulsierend angelegtes magnetisches Feld, wie oben beschrieben. Bspw. ist eine Ursache der Zunahme einer Partikeldeposition mit steigendem Atemminutenvolumen der dadurch ansteigende Inspirationsfluss (Fluss beim Einatmen). Auch führt eine End-inspiratorische (am Ende des Ausatmens erfolgende) Atemanhaltezeit zu einem Anstieg der Aerosoldeposition. Bei der Geschwindigkeit (des erfindungsgemäßen Magnetpartikel-haltigen Aerosols und der darin enthaltenen Magnetpartikel und pharmazeutischen Wirkstoffe gemäß der Erfindung) ist zwischen der Geschwindigkeit, mit der das erfindungsgemäße Magnetpartikel-haltige Aerosol verabreicht, d.h. vernebelt (und eingeatmet), wird, und der Geschwindigkeit, mit der sich das Aerosol nach Verabreichung in den Atemwegen bewegt, zu unterscheiden. Die Geschwindigkeit, mit der sich das Aerosol nach Verabreichung in den Atemwegen bewegt, ist insbesondere durch physiologische Faktoren, wie bspw. der Atemwegsgeometrie, z.B. dem Durchmesser des Atemweges des zu behandelnden Patienten, vorgegeben und beträgt z.B. an der zweiten Verzweigung des Atemwegs typischerweise etwa 4,7 m/s. Die Geschwindigkeit, mit der das Aerosol und damit die Magnetpartikel und pharmazeutischen Wirkstoffe der Erfindung verabreicht wird/werden, sollte vorteilhafterweise mindestens 3 m/s, vorzugsweise mindestens 5 m/s, stärker bevorzugt mindestens 8 m/s, noch stärker bevorzugt mindestens 10 m/s betragen, um einen effektiven Transport des erfindungsgemäßen Magnetpartikel-haltigen Aerosols *in vivo* zu gewährleisten. Der Fachmann wird in der Lage sein, unter Berücksichtigung der oben genannten Faktoren die jeweils geeignete Geschwindigkeit für die Verabreichung des erfindungsgemäßen Magnetpartikel-haltigen Aerosols festzulegen.

Neben den Magnetpartikeln enthält das erfindungsgemäße Magnetpartikel-haltige Aerosol mindestens einen pharmazeutischen Wirkstoff. Unter einem "pharmazeutischen Wirkstoff" ist gemäß der vorliegenden Erfindung jeder (klassische und neuartige) Arzneistoff zu verstehen, der zur Behandlung einer Erkrankung der Atemwege oder der Lunge geeignet ist. Unter "klassischen Arzneistoffen" sind insbesondere sog. "small drug" Arzneistoffe, d.h. niedermolekulare Arzneistoffe zu verstehen. Beispiele hierfür sind, ohne darauf beschränkt zu sein, Salbutamol, Formoterol, Ipatropiumbromid, Budesonid, Fenoterol, Terbutalin, Tiotropiumbromid, Salmeterol, Beclometason, Fluticason, Mometason, Ciclesonid, Natriumcromoglicat, Nedocromil-Dinatrium, Tobramycin, Theophyllin, Gentamycin, Paclitaxel und Camptothecin. Unter "neuartigen Arzneistoffen" sind insbesondere höhermolekulare Arzneistoffe zu verstehen, wie bspw. Zytostatika, Peptide, Proteine oder Nukleinsäuren sowie Broncholytika, Antibiotika, Antidiabetika und Immunmodulatoren. Bei den Peptiden und Proteinen kann es sich in diesem Zusammenhang bspw. um Dornase $\alpha$, Insulin, $\alpha$-1 Antitrypsin, Katalase, Superoxiddismutase, Interleukin-2, Surfactant Proteine, dem Sekretorischen Leukoprotease Inhibitor, Interferon-$\gamma$, IL-1R, Anti-IgE Mab (monoklonaler Antikörper), Calcitonin, Parathormon, Somatropin, Interfon-$\beta$, LH-RH Analoga, Ribaririn, Interferon-$\alpha$, rh-G-CSF, Erythropoietin, Heparin, 1-Deaminocystein-8-D-arginin-vasopression, Ricin-Vakzin und Cyclosporin handeln. Bei den zuvor beschriebenen Nukleinsäuren handelt es sich vorzugsweise um solche Nukleinsäuren, die zuvor genannten Peptide oder Proteine kodieren. Bevorzugt handelt es sich bei den Nukleinsäuren um DNA, vorzugsweise natürliche oder synthetische DNA, cDNA, genomische DNA, nackte DNA, einzelsträngige DNA, doppelsträngige DNA oder zirkuläre DNA, oder um RNA, vorzugsweise mRNA, ebenfalls bevorzugt RNAi, die in ihrer Länge keinen Einschränkungen unterliegen.

Der im erfindungsgemäßen Magnetpartikel-haltigen Aerosol enthaltene pharmazeutische Wirkstoff umfasst ebenfalls beliebige Zytostatika, die für die Behandlung von Atemwegs- und/oder Lungenerkrankungen geeignet sind. Unter "Zytostatika" sind vorliegend v.a. solche Verbindungen zu verstehen, die in allgemeiner Weise toxisch auf körpereigene Zellen wirken und so das Zellwachstum hemmen. Insbesondere sind hierbei Chemotherpeutika gegen Lungenkrebserkrankungen zu nennen. Je nach ihrem Wirkungsmechanismus werden Zytostatika verschiedener Gruppen unterschieden. Beispielhaft sind z.B. hier mit umfaßt:

- Alkylierende und quervernetzende Zytostatika, die die DNA schädigen. Beispiele hierfür sind Cyclophosphamid, N-Nitrosoverbindungen, wie Carmustin, Ethylenimin-(Aziridin-)Derivate, wie Thiotepa, Methansulfate, wie Busulfan, Platin-Komplexe, wie Cisplatin, Procarbazin und andere;
- Zytostatische Antibiotika, bspw. Anthracycline, wie Daunorubicin, Doxorubicin, Bleomycin und Mitomycine. Letztgenannte interkalieren in DNA und hemmen Topoisomerasen;
- Antimetabolite, die natürliche Stoffwechselbausteine, verdrängen. Beispiele sind Folsäure-Antagonisten, wie Methotrexat, Nucleosid-Analoga, wie Mercaptopurin, Fluorouracil und andere; und
- Hormone und Hormonantagonisten. Diese werden insbesondere bei Tumoren eingesetzt, deren Wachstum hormonabhängig ist. Beispiele sind (anti-) Östrogene, wie Formestan, Gestagene und Antiandrogene.

Die pharmazeutischen Wirkstoffe des Magnetpartikel-haltigen Aerosols der vorliegenden Erfindung können in einer vorformulierten Weise vorliegen, bspw. verpackt in geeigneten Mitteln zum Transport von pharmazeutischen Wirkstoffen, sog. "drug delivery" Systemen, bspw. in Nanopartikeln, Vektoren, bevorzugt Gentransfer-Vektoren, viralen oder nicht viralen Vektoren, Poly- oder Lipoplex-Vektoren, Liposomen oder einem in Hohlkolloid (d.h. Hohlkugeln kolloider Dimension). Es kann sich jedoch auch um nackte Nukleinsäuren, insbesondere nackte DNA, handeln. Geeignete Vektoren, Liposomen, Hohlkolloide oder Nanopartikel sowie Verfahren zum Einbringen von Substanzen, wie den erfindungsgemäßen pharmazeutischen Wirkstoffen, in solche Vektoren, Liposomen, Hohlkolloide oder Nanopartikel sind allgemein im Stand der Technik gut bekannt und bspw. in Cryan S-A. (Carrier-based Strategies for Targeting Protein and Peptide Drugs to the Lungs, AAPS Journal. 2005; 07((01): E20-E41) und in Sambrook et al. Molecular Cloning A Laboratory Manual, Cold Spring Harbor Laboratory (1989) NY beschrieben. Als Gentransfer-Vektoren können vorzugsweise Polyethylenimine oder kationische Lipide, wie z.B DOTAP, verwendet werden. Liposomen sind bevorzugt für die Verpackung von Zytostatika verwendbar (z.B. Dilauroylphosphatidylcholine); eine detaillierte Beschreibung erfolgt bspw. in Koshkina, N. V. et al. (Paclitaxel liposome aerosol treatment induces inhibition of pulmonary metastases in murine renal carcinoma model. Clinical Cancer Research 7, 3258-3262 (2001)). Proteine als pharmazeutische Wirkstoffe können vorzugsweise mittels superkritischer Flüssigkeiten, Emulsionsverfahren und Sprühtrocknung in biokompatible Poly-Milch/Glykolsäure-Polymere (PLGA) verpackt werden.

Ebenfalls können die pharmazeutischen Wirkstoffe des erfindungsgemäßen Magnetpartikel-haltigen Aerosols mit einer Magnetschicht beschichtet werden. Das Material einer solchen Magnetschicht besteht oder umfasst vorzugsweise aus einem der oben beschriebenen Materialen für Magnetpartikel. Verfahren für derartige Beschichtungen sind im Stand der Technik bekannt und gehören zum allgemeinen Fachwissen.

Die pharmazeutischen Wirkstoffe des Magnetpartikel-haltigen Aerosols der vorliegenden Erfindung können an die Magnetpartikel gebunden oder adsorbiert oder nicht an diese gebunden oder adsorbiert vorliegen. Im Fall einer Bindung oder Adsorption des/der pharmezeutische(n) Wirkstoff(e) an die Magnetpartikel, kann die Bindung physikalischer oder chemischer Art sein oder auf biologischer Interaktion beruhen. Solche Bindung bzw. Adsorption schließt z.B. elektrostatische, hydrophobe oder hydrophile Wechselwirkungen, Van-der-Waals-Wechselwirkungen, Wasserstoffbrückenbindugen und kovalente Bindungen ein. Bevorzugte kovalente Bindungen sind bspw. Amid-, Ester-, Thioester-, Ether-, Thioether- und Disulfidbindungen. Ebenfalls sind sämtliche Kombinationen der genannten Wechselwirkungen umfasst. Die Bindung zwischen Magnetpartikel und pharmazeutischem Wirkstoff im erfindungsgemäßen Magnetpartikel-haltigen Aerosol kann bspw. durch eine Reaktion, z.B. eine chemische Kopplung, von funktionalen Gruppen der Beschichtung des Magnetpartikels, wie oben beschrieben, und funktionalen Gruppen eines hier beschriebenen Vektors erfolgen. Alternativ kann die Bindung auch durch Verwenden eines (homo- oder heterobifunktionalen) Linkers erfolgen. Solche geeigneten homo- oder heterobifunktionalen Linker sind kommerziell erhältlich. Verfahren, die für eine (chemische) Kopplung von Magnetpartikeln und pharmazeutischen Wirkstoffen der vorliegenden Erfindung, z.B. unter Verwendung von wie oben beschriebenen Linkern oder funktionalen Gruppen, verwendbar sind, sind im Stand der Technik gut bekannt und bspw. ausführlich in Bioconjugate Techniques, von Greg T. Hermanson Academic Press (1. Januar 1996) beschrieben.

Sofern die Komponenten des erfindungsgemäßen Magnetpartikel-haltigen Aerosols, d.h. Magnetpartikel und pharmazeutische(r) Wirkstoff(e), gebunden vorliegen, können diese sowohl in Verbindung mit einem erfindungsgemäßen Lösungsmittel als auch ohne ein solches vorliegen. Im erstgenannten Fall handelt es sich gemäß der vorliegenden Erfindung bei den Magnetpartikel-haltigen Aerosolen um flüssige Aerosole, im letztgenannten Fall um trockene Aerosole. Besonders bevorzugt ist ein erfindungsgemäßes Magnetpartikel-haltiges Aerosol, in dem Magnetpartikel und pharmazeutische(r) Wirkstoff(e), gebunden vorliegen, in Verbindung mit einem wie im folgenden beschriebenen Lösungsmittel.

Die Komponenten des erfindungsgemäßen Magnetpartikel-haltigen Aerosols, d.h. der enthaltene Magnetpartikel und der pharmazeutische Wirkstoff, können im erfindungsgemäßen Magnetpartikel-haltigen Aerosol auch ungebunden vorliegen. Um in einem solchen erfindungsgemäßen Magnetpartikel-haltigen Aerosol eine gerichtete Deponierung des Wirkstoffs zu ermöglichen, wird bevorzugt ein wie im folgenden beschriebenes Lösungsmittel zusammen mit dem Magnetpartikel und dem pharmazeutischen Wirkstoff eingesetzt. Erfindungsgemäß handelt es sich dann auch hier um flüssige Aerosole. Der Transport erfolgt in diesem Fall mittels des Flüssigkeitstropfens, in dem sowohl (der) Magnetpartikel und (der) pharmazeutische(r) Wirkstoff(e) enthalten (ist) sind, wobei erst der enthaltene, Magnetpartikel den gerichteten Transport und die zielgerichtete Deponierung des Wirkstoffs ermöglicht. Erhalten werden solche Flüssigkeitstropfen typischerweise durch einen Vernebler oder eine äquivalente Vorrichtung, wie nachfolgend für die Herstellung erfindungsgemäßer (flüssiger) Aerosole beschrieben. Besonders bevorzugt ist daher ebenfalls ein erfindungsgemäßes Magnetpartikel-haltiges Aerosol, in dem Magnetpartikel und pharmazeutische(r) Wirkstoff(e), ungebunden vorliegen, in Verbindung mit einem wie im folgenden beschriebenen Lösungsmittel.

Bei einem für ein erfindungsgemäßes Magnetpartikel-haltiges Aerosol verwendbares Lösungsmittel kann es sich um ein anorganisches oder organisches Lösungsmittel handeln. Bevorzugte Lösungsmittel sind Ethanol, Wasser und Glycerin (Glycerol) oder Mischungen hiervon. Gemäß der vorliegenden Erfindung geeignete Lösungsmittel sollten vorzugsweise von dem Lebewesen (Säugetier, vorzugsweise Mensch), dem das Aerosol verabreicht wird, physiologisch gut verträglich sein, d.h. keine Nebenwirkungen, z.B. toxische Nebenwirkungen, auslösen. Ein insbesondere bevorzugtes Lösungsmittel ist destilliertes Wasser. Ebenfalls bevorzugt sind Ethanol-Wasser-Mischungen; hierbei liegt der prozentuale Massenanteil von Ethanol in diesen Mischungen bevorzugt in einem Bereich zwischen 5 % und 99 % Ethanol, ebenfalls bevorzugt im Bereich von 10 % bis 96 % Ethanol, stärker bevorzugt zwischen 50 % und 92 %, am stärksten bevorzugt zwischen 69 % und 91 % Ethanol.

Das Herstellen eines erfindungsgemäßen, Lösungsmittel enthaltenden, Magnetpartikel-haltigen Aerosols mit einem nicht an die Magnetpartikel gebundenen pharmazeutischen Wirkstoffs kann bspw. durch Herstellen von Flüssigkeitstropfen, bestehend aus einem oben beschriebenen Lösungsmittel und darin enthaltener Magnetpartikel sowie einem pharmazeutischen Wirkstoff (nachfolgend erfindungsgemäße Formulierung genannt) erfolgen. Hierzu werden die genannten Komponenten gemischt und die Flüssigkeitstropfen bspw. mit einem Vernebler, (siehe unten) oder einer äquivalenten Vorrichtung, erzeugt. Sofern der pharmazeutische Wirkstoff an die Magnetpartikel gebunden wird, erfolgt dies vor dem Mischen der genannten Komponenten und somit auch vor dem Erzeugen der Flüssigkeitstropfen aus der erfindungsgemäßen Formulierung.

Die Herstellung erfindungsgemäßer Magnetpartikel-haltiger Aerosole ohne Lösungsmittel kann durch Mischen der Komponenten Magnetpartikel und pharmazeutischer Wirkstoff erfolgen. Ggf. wird dazu der pharmazeutische Wirkstoff vorab an die Magnetpartikel gebunden und nachfolgend einer Behandlung mit einem geeigneten Vernebler, einem Treibgasvernebler (siehe unten) oder einer äquivalenten Vorrichtung, erfolgen.

Die Herstellung der erfindungsgemäßen Aerosole kann jedoch ebenfalls durch jedes geeignete Verfahren aus dem Stand der Technik erfolgen.

Ein weiterer Gegenstand der vorliegenden Erfindung ist eine pharmazeutische Zusammensetzung, die ein erfindungsgemäßes Magnetpartikel-haltiges Aerosol sowie gegebenenfalls geeignete Hilfs- und/oder Zusatzsstoffe enthält. Vorzugsweise enthält die pharmazeutische Zusammensetzung weiterhin mindestens ein (weiteres) Lösungsmittel, mindestens einen Komplexbildner und/oder mindestens eine pharmazeutisch verträgliche Säure.

Unter erfindungsgemäßen "Hilfs- und/oder Zusatzsstoffen" ist im Zusammenhang mit der erfindungsgemäßen pharmazeutischen Zusammensetzung jede pharmakologisch verträgliche und therapeutisch sinnvolle Substanz zu verstehen, die kein pharmazeutischer Wirkstoff ist, jedoch zusammen mit dem pharmazeutischen Wirkstoff in der pharmazeutischen Zusammensetzung formuliert werden kann, um qualitative Eigenschaften der pharmazeutischen Zusammensetzung zu beeinflussen, insbesondere zu verbessern. Bevorzugt entfalten die Hilfs- und/oder Zusatzsstoffe keine oder im Hinblick auf die beabsichtigte Therapie keine nennenswerte oder zumindest keine unerwünschte pharmakologische Wirkung. Geeignete Hilfs- und Zusatzsstoffe sind bspw. pharmakologisch unbedenkliche Salze, bspw. Natriumchlorid, Geschmacksstoffe, Vitamine, z.B. Vitamin A oder Vitamin E, Tocopherole oder ähnliche im menschlichen Organismus vorkommende Vitamine oder Provitamine, Antioxidantien, wie bspw. Ascorbinsäure, sowie Stabilisatoren und/oder Konservierungsstoffe zum Verlängern der Verwendungs- und Aufbewahrungsdauer der pharmazeutischen Zusammensetzung und sonstige im Stand der Technik bekannte übliche Hilfs- und Zusatzstoffe.

Konservierungsstoffe können in der erfindungsgemäßen pharmazeutischen Zusammensetzung bspw. eingesetzt werden, um die pharmazeutische Zusammensetzung vor Kontaminationen mit pathogenen Keimen zu schützen. Als Konservierungsstoffe eignen sich insbesondere Benzalkoniumchlorid oder Benzoesäure bzw. Benzoate wie Natriumbenzoat in den aus dem Stand der Technik bekannten Konzentrationen. Die Menge von bspw. zugesetztem Benzalkoniumchlorid beträgt bevorzugt ungefähr 1 mg bis 50 mg pro 100 ml, stärker bevorzugt ungefähr 7 mg bis 15 mg pro 100 ml, noch stärker bevorzugt ungefähr 9 mg bis 12 mg pro 100 ml der erfindungsgemäßen pharmazeutischen Zusammensetzung. Besonders bevorzugt sind jedoch erfindungsgemäße pharmazeutischer Zusammensetzung, die keine Konservierungsstoffe enthalten.

Unter dem mindestens einen (weiteren) "Lösungsmittel" der erfindungsgemäßen pharmazeutischen Zusammensetzung sind die oben im Zusammenhang mit dem erfindungsgemäßen Magnetpartikel-haltigen Aerosol beschriebenen Lösungsmittel zu verstehen. Sofern dabei in der erfindungsgemäßen pharmazeutischen Zusammensetzung erfindungsgemäße flüssige Magnetpartikel-haltige Aerosole eingesetzt werden, ist das mindestens eine (weitere) "Lösungsmittel" mindestens ein zusätzliches zu dem im flüssigen Aerosol enthaltenen Lösungsmittel. Bei erfindungsgemäßen flüssigen Magnetpartikel-haltigen Aerosolen ist das mindestens eine (weitere) "Lösungsmittel" entsprechend mindestens ein "Lösungsmittel". Das in der erfindungsgemäßen pharmazeutischen Zusammensetzung enthaltene mindestens eine (weitere) "Lösungsmittel" kann ein Lösungsmittel oder jede beliebige Kombination der oben beschriebenen Lösungsmittel enthalten.

Unter "Komplexbildner" im Zusammenhang mit der erfindungsgemäßen pharmazeutischen Zusammensetzung sind Moleküle zu verstehen, die geeignet sind, Komplexbindungen einzugehen. Bevorzugt werden durch diese Verbindungen erfindungsgemäß Kationen, besonders bevorzugt metallische Kationen, komplexiert. Bevorzugte Komplexbildner sind Editinsäure (EDTA, Ethylendiamintetraacetat) oder ein bekanntes Salze davon, z. B. Natrium-EDTA oder Dinatrium-EDTA. Bevorzugt wird Natriumedetat (Ethylendiamintetraessigsäure Dinatriumsalz), gegebenenfalls in Form seiner Hydrate, besonders bevorzugt in Form seines Dihydrats eingesetzt. Die Konzentration der eingesetzten Komplexbildner liegt bevorzugt in einem Bereich von ungefähr 1 mg bis 100 mg pro 100 ml, stärker bevorzugt in einem Bereich von ungefähr 5 mg bis 50 mg pro 100 ml, stärker bevorzugt von ungefähr 6 mg bis 30 mg pro 100 ml, am stärksten bevorzugt von ungefähr 7 mg bis 20 mg pro 100 ml der erfindungsgemäßen pharmazeutischen Zusammensetzung.

Pharmazeutisch verträgliche anorganische oder organische Säuren werden insbesondere zur Einstellung des pH-Wertes der erfindungsgemäßen pharmazeutischen Zusammensetzung verwendet, wobei dieser pH-Wert bevorzugt in einem Bereich von ungefähr 3,0 bis ungefähr 8,5, bevorzugt zwischen 3,5 und 6,0, besonders bevorzugt zwischen 4,0 und 7,0 liegt. Ganz besonders bevorzugt beträgt der pH-Wert bei etwa 7,0. Pharmazeutisch verträgliche anorganische oder organische Säuren können grundsätzlich aus allen geeigneten Säuren oder Basen ausgewählt werden. Beispiele für bevorzugte (pharmazeutisch verträgliche) anorganische Säuren können im Zusammenhang mit der erfindungsgemäßen pharmazeutischen Zusammensetzung ausgewählt werden aus der Gruppe bestehend aus Salzsäure, Bromwasserstoffsäure, Salpetersäure, Schwefelsäure und Phosphorsäure, wobei insbesondere Salzsäure und Schwefelsäure bevorzugt sind. Beispiele für besonders geeignete (pharmazeutisch verträgliche) organische Säuren können im Zusammenhang mit der erfindungsgemäßen pharmazeutischen Zusammensetzung ausgewählt werden aus der Gruppe bestehend aus Äpfelsäure, Weinsäure, Maleinsäure, Bernsteinsäure, Essigsäure, Ameisensäure und Propionsäure und insbesondere bevorzugt Ascorbinsäure, Fumarsäure und Zitronensäure. Gegebenenfalls können auch Mischungen der genannten Säuren eingesetzt werden, insbesondere von Säuren, die neben ihren Säuerungseigenschaften auch andere Eigenschaften, z.B. in Verwendung als Geschmackstoffe oder Antioxidantien, besitzen, wie bspw. Zitronensäure oder Ascorbinsäure.

Weiterhin können zum genauen Einstellen des oben beschriebenen pH-Wertes der erfindungsgemäßen pharmazeutischen Zusammensetzung gegebenenfalls auch pharmazeutisch verträgliche Basen eingesetzt werden. Als Basen eignen sich in diesem Zusammenhang Alkalihydroxide, Alkalicarbonate und Alkali-Ionen, bevorzugt Natrium. Es ist zu beachten, dass auch die aus solchen Basen resultierenden Salze, die dann in der erfindungsgemäßen pharmazeutischen Zusammensetzung enthalten sind, mit der/den oben genannte(n) Säure pharmazeutisch verträglich sind. Die Kombination der jeweils geeigneten Säuren und Basen zum Einstellen des pH-Wertes der erfindungsgemäßen pharmazeutischen Zusammensetzung kann je nach Bedarf und den Erfordernissen der herzustellenden Zusammensetzung variieren. Eine entsprechende Auswahl oder Kombination solcher, wie oben beschriebenen, Säuren und Basen kann leicht durch einen Fachmann durchgeführt werden.

Die Herstellung einer erfindungsgemäßen pharmazeutischen Zusammensetzung kann analog zu der oben beschriebenen Herstellung der erfindungsgemäßen Aerosole erfolgen. Gegebenenfalls enthaltene weitere Komponenten der erfindungsgemäßen pharmazeutischen Zusammensetzung, d.h. Hilfs- und/oder Zusatzsstoffe, das vorzugsweise enthaltene Lösungsmittel, Komplexbildner und/oder mindestens eine pharmazeutisch verträgliche Säure können nach im Stand der Technik bekannten Verfahren im oben beschrieben Aerosol-Herstellungsverfahren zugesetzt werden.

Alternativ kann die erfindungsgemäße pharmazeutische Zusammensetzung durch jedes beliebige im Stand der Technik bekannte Verfahren erfolgen.

Die Dosierung der zuvor beschriebenen Komponenten der erfindungsgemäßen pharmazeutischen Zusammensetzung unterliegt verschiedenen Faktoren, bspw. der Art der Behandlung, Erkrankung, Zustand des Lebewesens (Säugetier, vorzugsweise Mensch), dem Alter des erkrankten Lebewesens, dem die erfindungsgemäße pharmazeutische Zusammensetzung verabreicht wird, der Art des Wirkstoffs etc.. Dem Fachmann sind solche Parameter bekannt, die Bestimmung der Dosierungen unterliegt seinem allgemeinen Fachwissen.

Das erfindungsgemäße Magnetpartikel-haltige Aerosol sowie die erfindungsgemäße pharmazeutische Zusammensetzung dienen insbesondere der nasalen oder oralen Verabreichung. Eine bevorzugte Ausführungsform betrifft daher ein erfindungsgemäßes Magnetpartikel-haltiges Aerosol oder eine erfindungsgemäße pharmazeutische Zusammensetzung zur nasalen oder oralen Verabreichung. Die Verabreichung erfolgt bevorzugt über einen Vernebler. Daher betrifft eine weitere bevorzugte Ausführungsform ein erfindungsgemäßes Magnetpartikel-haltiges Aerosol oder eine erfindungsgemäße pharmazeutische Zusammensetzung zur Verabreichung über einen Vernebler.

Ein "Vernebler" im Sinne der vorliegenden Erfindung ist jeder beliebige für medizinische Aerosole geeignete Standardvernebler. Der Begriff "Vernebler" ist synonym mit dem Begriff "Inhalator" zu verstehen. Vernebler werden üblicherweise verwendet, um erfindungsgemäße flüssige und/oder trockene Magnetpartikel-haltige Aerosole, z.B. auf der Basis von Lösungsmitteln (wie oben bereits beschrieben), herzustellen und zu verabreichen. Dazu werden üblicherweise die erfindungsgemäßen Formulierungen einem Vernebler zugeführt, um aus dieser die erfindungsgemäßen, bevorzugt treibgasfreien, Magnetpartikel-haltigen Aerosole herzustellen. Der Vernebler versprüht hierzu typischerweise ein definiertes Volumen der Formulierung unter Anwendung hoher Drücke durch kleine Düsen, um so ein inhalierbares erfindungsgemäßes Magnetpartikel-haltiges Aerosol zu erzeugen. Besonders geeignet sind hierbei Vernebler, die eine kleine Menge einer flüssigen erfindungsgemäßen Formulierung in der therapeutisch notwendigen Dosierung binnen weniger Sekunden in ein therapeutisch-inhalativ geeignetes Aerosol vernebeln können. Solche Vernebler sind insbesondere für eine Treibgas-freie Verabreichung der erfindungsgemäßen (flüssigen) Magnetpartikel-haltigen Aerosole oder pharmazeutischen Zusammensetzungen geeignet. Ein derartige Vernebler ist z.B. in den internationalen Patentanmeldungen WO 91/14468 und WO 97/12687 beschrieben, In einem solchen Vernebler wird eine Arzneimittellösung mittels hohen Drucks von bis zu 600 bar in ein medizinisches, für die Applikation an Atemwege und Lunge geeignetes, Aerosol überführt und versprüht. Zur Vernebelung einer solchen Lösung wird eine spezielle Düse verwendet, wie sie bspw. die WO 94/07607 oder die WO 99/16530 beschribt.

Geeignete Vernebler zur nasalen oder oralen Verabreichung der erfindungsgemäßen trockenen Magnetpartikel-haltigen Aerosole, d.h. erfindungsgemäße Magnetpartikel-haltige Aerosole ohne ein Lösungsmittel, sind typischerweise Treibgas-getriebene Inhalationsgeräte bzw. Vernebler. Treibgase können hierbei bspw. FCKW oder HFKW sein. Diesbezüglich wird auf die Druckschrift "Theorie und Praxis der Inhalationstherapie", Seiten 31 bis 70, Arcis Verlag (2000) verwiesen, in der eine ausführliche Beschreibung verwendbarer Vernebler sowie Verfahren zu deren Anwendung offenbart ist/sind.

Weitere Beispiele für geeignete Vernebler zur nasalen oder oralen Verabreichung der erfindungsgemäßen Magnetpartikel-haltigen Aerosole sind pressluftgetriebene Düsenvernebler (z.B. PARI LC plus, PARI GmbH, Starnberg, Deutschland), Venturi-Düsenvernebler, Wasserdampfgetriebene Düsenvernebler oder Ultraschallvernebler (z.B. AeronebLab, Aerogen, Inc., Stierlin Court, Canada; eFLOW, PARI GmbH, Starnberg, Deutschland). Ebenfalls geeignet sind Vernebler mit einer Größe, die von dem Patienten (Mensch) mitgeführt werden können, z.B. der Respimat@, wie in WO 97/12687 beschrieben. Eine ausführliche Beschreibung geeigneter Vernebler findet sich auch in "Theorie und Praxis der Inhalationstherapie", Seiten 31 bis 70, Arcis Verlag (2000).

Ein weiterer Gegenstand der vorliegenden Erfindung ist daher auch eine Vorrichtung zur Vernebelung und/oder Verabreichung des erfindungsgemäßen Magnetpartikel-haltigen Aerosols, enthaltend einen wie oben beschriebenen Vernebler, befüllt mit einem erfindungsgemäßen Magnetpartikel-haltigen Aerosol. Die erfindungsgemäße Vorrichtung kann als Vorrichtung mit einem auswechselbaren Vorrat an erfindungsgemäßem Magnetpartikel-haltigen Aerosol ausgeführt sein. Alternativ kann die erfindungsgemäße Vorrichtung eine Einweg-Vorrichtung sein. Die erfindungsgemäße Vorrichtung kann weiterhin mobil oder stationär sein. Falls die erfindungsgemäße Vorrichtung mobil ist, beträgt das Gewicht bevorzugt nicht mehr als 2 kg, besonders bevorzugt nicht mehr als 1 kg, noch stärker bevorzugt nicht mehr als 500 g. Falls die erfindungsgemäße Vorrichtung stationär ausgeführt ist, umfasst die Vorrichtung bevorzugt einen auswechselbaren Vorrat an erfindungsgemäßem Magnetpartikel-haltigen Aerosol.

Ein weiterer Gegenstand der vorliegenden Erfindung ist die Verwendung eines erfindungsgemäßen Magnetpartikel-haltigen Aerosols oder einer erfindungsgemäßen pharmazeutischen Zusammensetzung zur Prophylaxe und/oder Therapie von Atemwegs- und/oder Lungenerkrankungen, insbesondere von entzündlichen und/oder obstruktiven Aternwegs- und/oder Lungenerkrankungen, bspw. Melanomen oder malignen Melanomen der Atemwege, insbesondere der Lunge, Lungenkrebs, Lungentumoren, Lungenkarzinomen, kleinzelligen Lungenkarzinomen, Rachenkrebs, Bronchialkarzinomen, Kehlkopfkrebs, Kopf-Hals-Tumoren, Zungenkrebs, Sarkomen und Blastomen im Bereich oder Nähe der Atemwege, insbesondere der Lunge, sowie Asthma und COPD ("chronic obstructive pulmonary disease"; chronisch obstruktive Lungenerkrankung), Lungenemphysem, chronischer Bronchitis, Lungenentzündung sowie von Erbkrankheiten, bspw. Mukovizidose, humanem Surfactant Protein B-Mangel und $\alpha_1$-Antitrypsinmangel, sowie zur Therapie nach einer Lungentransplantation sowie zur pulmonalen Vakzininierung und zur antiinfektiven Therapie der Lunge (bei Bakterien- (z.B. *Bronchiolitis obliterans*) oder Virenbesiedelung der Lunge).

Ein weiterer Gegenstand der vorliegenden Erfindung ist die Verwendung eines erfindungsgemäßen Magnetpartikel-haltigen Aerosols oder ggf. einer erfindungsgemäßen pharmazeutischen Zusammensetzung zur Herstellung eines Arzneimittels oder einer Vorrichtung zur Prophylaxe und/oder Therapie von Atemwegs- und/oder Lungenerkrankungen, insbesondere von entzündlichen und/oder obstruktiven Atemwegs- und/oder Lungenerkrankungen, bspw. Melanomen oder malignen Melanomen der Atemwege, insbesondere der Lunge, Lungenkrebs, Lungentumoren, Lungenkarzinomen, kleinzelligen Lungenkarzinomen, Rachenkrebs, Bronchialkarzinomen, Kehlkopfkrebs, Kopf-Hals-Tumoren, Zungenkrebs, Sarkomen und Blastomen im Bereich oder Nähe der Atemwege, insbesondere der Lunge, sowie Asthma COPD, Lungenemphysem, chronischer Bronchitis, Lungenentzündung sowie von Erbkrankheiten, bspw. Mukoviszidose, humanem Surfactant Protein B-Mangel und $\alpha_1$-Antitrypsinmangel, sowie zur Therapie nach einer Lungentransplantation sowie zur pulmonalen Vakzininierung und zur antiinfektiven Therapie der Lunge (bei Bakterien- (z.B. *Bronchiolitis obliterans*) oder Virenbesiedelung der Lunge).

Ein anderer Gegenstand der vorliegenden Erfindung betrifft die Verwendung eines erfindungsgemäßen Magnetpartikel-haltigen Aerosols oder einer erfindungsgemäßen pharmazeutischen Zusammensetzung in der Diagnostik. Alternativ betrifft ein Gegenstand der vorliegenden Erfindung die Verwendung eines erfindungsgemäßen Magnetpartikel-haltigen Aerosols oder einer erfindungsgemäßen pharmazeutischen Zusammensetzung zur Herstellung einer Vorrichtung in der Diagnostik. Beispielsweise kann das Diagnostikum ein Kontrastmittel sein, umfassend z.B. jodhaltige (wasserlösliche) röntgenpositive Kontrastmittel; jodhaltige (wasserunlösliche) röntgenpositive Kontrastmittel; Barium-haltige (wasserunlösliche) röntgenpositive Kontrastmittel; Röntgen negative Kontrastmittel; Gadolinium-Verbindungen, wie z.B. Gadolinium-DTPA; Technetium-Verbindungen, wie z.B. 99m-Technetium-enthaltende Verbindungen, bspw. Technetium-99m markierte Phosphonate, Tc-99m-MDP = Tc-99m-Methylen-Diphosphonat, Tc-99m-Tetrofosmin; wie oben beschriebene superparamagnetische Eisenoxidpartikel; membrangängige Kontrastmittel; enzymspezifische NMR-Sonden, z.B. mit paramagnetischen dinuklearen Komplexen mit Lanthanoidionen auf der Basis von cyclischen Polyaminopolyessigsäuren; Glukose-Kontrastmittel, bspw. zur Verwendung in der PET (Positronen-Emissions-Tomographie), umfassend z.B. Glukose-Kontrastmittel, gebunden an eine markierte Substanz, die eine externe Lokalisierung ermöglicht, z.B. durch Verwendung von Isotopen, die Positronen aussenden, z.B. [18]Fluor, wie etwa [18]Fluor markierter Fluor-2-deoxy-2-D-Glukose (18FDG), etc.. Ferner kann das Diagnostikum Peptid, Proteine, Nukleinsäure; Antikörper, insbesondere detektierbare Antikörper, niedermolekulare detektierbare Verbindungen, etc. umfassen. Das erfindungsgemäße Magnetpartikel-haltige Aerosol oder eine erfindungsgemäße pharmazeutische Zusammensetzung kann zur Diagnostik durch den beschriebenen gerichteten Transport in definierte Bereiche der Atemwege, insbesondere der Lunge, eingebracht werden und in diesen Bereichen als Diagnostikum fungieren. Hierdurch können strukturelle Veränderungen der Lunge, die auf eine pathologische Veränderung hinweisen, frühzeitig erkannt und bildlich dargestellt werden und damit die Diagnose einer potentiellen Erkrankung erleichtern. Ein solches Diagnostikum ist insbesondere für die Bildgebung von Lungentumoren oder Lungenemphysemen (Bildgebung MRI, CT, Bestimmung des Ausmaßes eines Lungenemphysems) verwendbar.

Eine bevorzugte Ausführungsform der vorliegenden Erfindung betrifft die Verwendung eines erfindungsgemäßen Magnetpartikel-haltigen Aerosols oder einer erfindungsgemäßen pharmazeutischen Zusammensetzung zur gerichteten Aerosoldeposition in den Atemwegen oder in der Lunge eines Lebewesens (Säugetier, bevorzugt Mensch) sowie die Verwendung eines erfindungsgemäßen Magnetpartikel-haltigen Aerosols oder einer erfindungsgemäßen pharmazeutischen Zusammensetzung vorzugsweise zur nasalen oder oralen Verabreichung, vorzugsweise über einen Vernebler.

Ein weiterer Gegenstand der vorliegenden Erfindung ist ein Verfahren zur inhalativen Anwendung eines erfindungsgemäßen Magnetpartikel-haltigen Aerosols oder einer erfindungsgemäßen pharmazeutischen Zusammensetzung, wobei das Verfahren das Herstellen eines erfindungsgemäßen Magnetpartikel-haltigen Aerosols oder einer erfindungsgemäßen pharmazeutischen Zusammensetzung sowie das Verabreichen dieser an ein Lebewesen (Säugetier, vorzugsweise Mensch) umfasst. Die Herstellung und Verabreichung erfolgen bevorzugt wie vorangehend beschrieben.

Bevorzugt erfolgt das Verfahren zur inhalativen Anwendung eines erfindungsgemäßen Magnetpartikel-haltigen Aerosols unter Verwendung eines wie oben beschriebenen extern angelegten magnetischen Feldes, das permanent, oder noch stärker bevorzugt, nicht permanent aktiv ist. Besonders bevorzugt ist während der Verabreichung, das extern angelegte magnetische Feld lediglich während des Zeitraumes der Ruhephasen zwischen Einatmung und Ausatmung oder zwischen Ausatmung und Einatmung aktiv. Noch bevorzugter kann die Steuerung des Magnetfeldes in Koordination mit der Atmung des Patienten dynamisch in Abhängigkeit des Atemrhythmus des Patienten erfolgen, so dass während des Ein- und Ausatmens des Patienten im zu therapierenden Bereich kein Magnetfeld anliegt, jedoch in den Ruhephasen dort ein Magnetfeld anliegt und nur dann eine Deposition des erfindungsgemäßen Magnetpartikei-haltigen Aerosols auf der Oberfläche der Atemwege erfolgt. Bevorzugt ist dazu die Luft über der ausgewählten Oberfläche der Atemwege durch Verabreichung des erfindungsgemäßen Magnetpartikel-haltigen Aerosols derart gesättigt, daß innerhalb von mehreren Aktivierungen des Magnetfeldes/der Magnetfelder durch den/die Magneten eine signifikante Deposition des transportierten Wirkstoffs bzw. des erfindungsgemäßen Magnetpartikel-haltigen Aerosols auf der ausgewählten Oberfläche der Atemwege ermöglicht wird. Die Steuerung des Magneten kann bspw. durch eine elektrische Schaltung ermöglicht werden, die am Anfang und Ende der Einatmung bzw. Ausatmung des Patienten im Vernebler oder Inhalator ein Signal auslöst, durch das wiederum das Magnetfeld eines wie hier beschriebenen Magneten an- bzw. abgeschaltet wird.

Ein weiterer Gegenstand der vorliegenden Erfindung ist ein Kit, enthaltend ein erfindungsgemäßes Magnetpartikel-haltiges Aerosol oder eine erfindungsgemäße pharmazeutische Zusammensetzung, einen ein Magnetfeld erzeugenden externen Magneten sowie einen Vernebler.

Sämtliche in der Beschreibung der vorliegenden Erfindung aufgeführten Referenzen werden vollumfänglich in die vorliegende Erfindung eingeschlossen. Nachfolgend wird die vorliegende Erfindung anhand von Figuren und Beispielen näher dargestellt. Es ist nicht beabsichtigt, die vorliegende Erfindung hierauf zu beschränken.

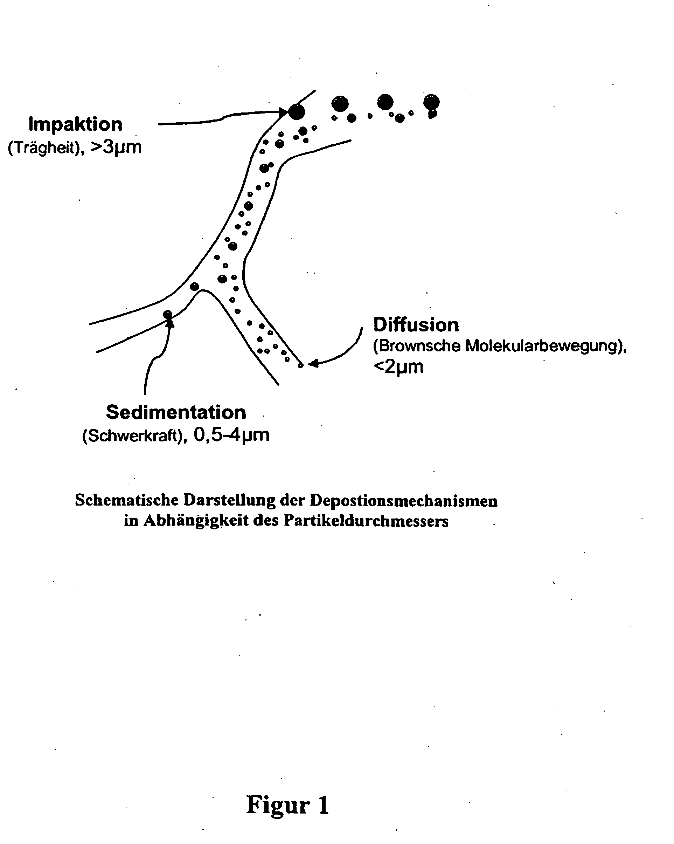

**Figur 1**    stellt die verschiedenen Mechanismen der Deposition von Aerosolpartikeln in der Lunge dar. Dargestellt ist die hypothetische Deposition an der Luftrauminnenwand (Atemwegsepithel) verschiedener Partikel in Abhängigkeit ihres Durchmessers und des daraus folgenden Depositionsmechanismus.

**Figur 2**    zeigt die normale lokale Deposition von Aerosolpartikeln in Abhängigkeit ihres Durchmessers in der Lunge bei ruhiger Nasenatmung. Untersuchungen bzgl. der lokalen Deposition haben ergeben, dass der Partikeldurchmesser einen entscheidenden Einfluss auf die lokale Deposition hat.

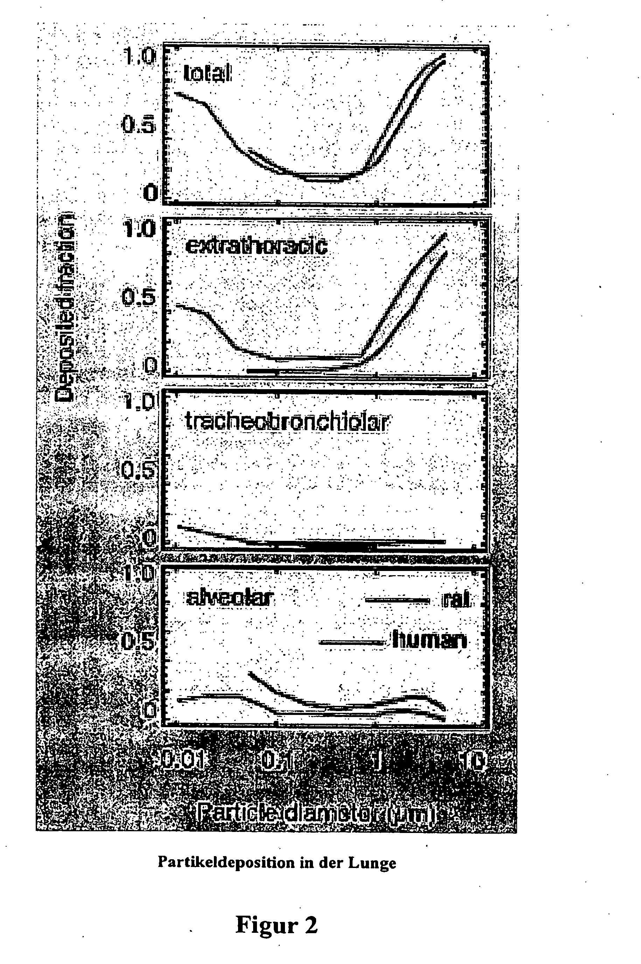

Aus Figur 2 ist ersichtlich, dass inhalierte Partikeln mit einem Durchmesser zwischen 0,1 bis 1 $\mu$m insgesamt nur minimal in der Lunge deponiert werden. Dagegen steigt die Deposition von Partikeln mit größerem und kleinerem Durchmesser insgesamt an. Aus Figur 2 ist ebenfalls ersichtlich, dass Partikel mit einem Durchmesser von 0,01 $\mu$m zu ca. 80% und 5 $\mu$m zu ca. 100% deponiert werden. Die ansteigende Deposition größerer Partikel ist vor allem auf das mit zunehmender Partikelgröße ansteigende Abscheiden durch Sedimentation und Impaktion zurückzuführen. Im Gegensatz dazu nimmt die Deposition kleiner Partikel < 0,1 $\mu$m aufgrund des Abscheidens durch Diffusion zu. Die Gesamtdeposition während ruhiger Nasenatmung ist bei der Ratte und beim Menschen vergleichbar.

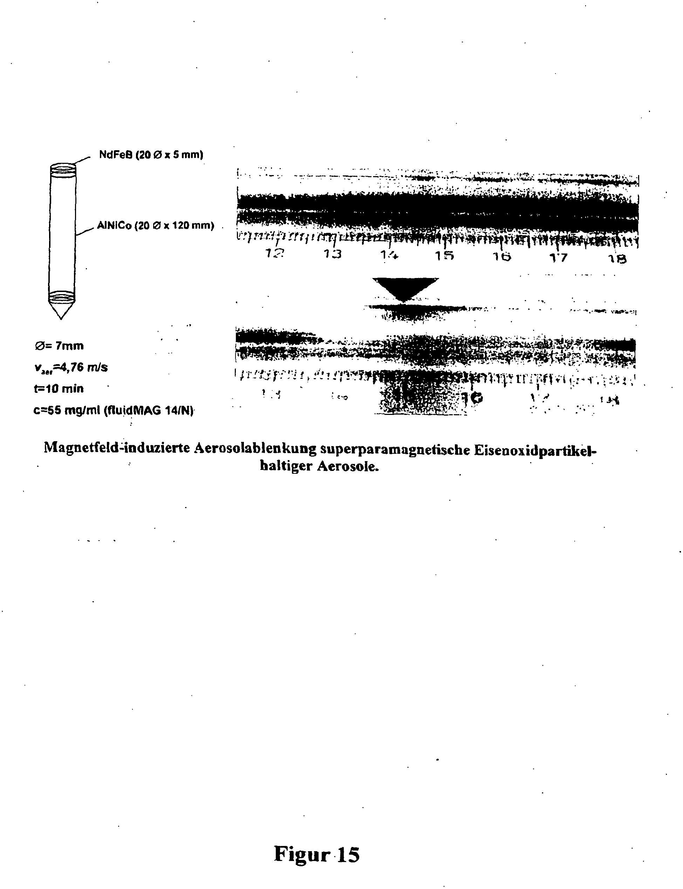

Erfindungsgemäß wurde gezeigt, dass die Deposition von Partikeln durch die erfindungsgemäße Magnetfeld-geteuerte Deposition erheblich erhöht werden konnte (siehe auch Figur 15).

Hinsichtlich der lokalen Deposition der Partikel zeigt Figur 2, dass auch diese stark von dem Partikeldurchmesser abhängt. Die extrathorakale Deposition erfolgt hauptsächlich in der Nase und ist nur geringfügig niedriger als die Gesamtdeposition. Insbesondere Partikel mit einem Durchmesser größer als 1 $\mu$m oder kleiner als 0,05 $\mu$m, werden in der Nase bereits gefiltert. Der restliche Anteil des inhalierten Aerosols wird größtenteils im Alveolargebiet deponiert. Der Aerosolanteil, der in der tracheobronchialen Region (Luftröhre und Atemwege) deponiert, ist relativ niedrig, und erreicht für sehr kleine Partikel bis zu 15 %, während Partikel > 3 $\mu$m nur zu 1 bis 3 % deponiert werden. In der Alveolarregion werden ca. 15 % der inhalierten Dosis von Partikeln eines Durchmessers < 0,1 $\mu$m deponiert. Für Partikel mit einem Durchmesser von 0,1 bis 3 $\mu$m variiert der deponierte Anteil zwischen 5 bis 10 %. Die Deposition ist fast vernachlässigbar für Partikel > 5 $\mu$m. Ingesamt lässt sich feststellen, dass die tracheobronchiale Deposition zwischen der Ratte und dem Menschen sehr ähnlich ist (siehe Schulz, H. & Muhle, H. 323-345 (Academic Press, 2000)).

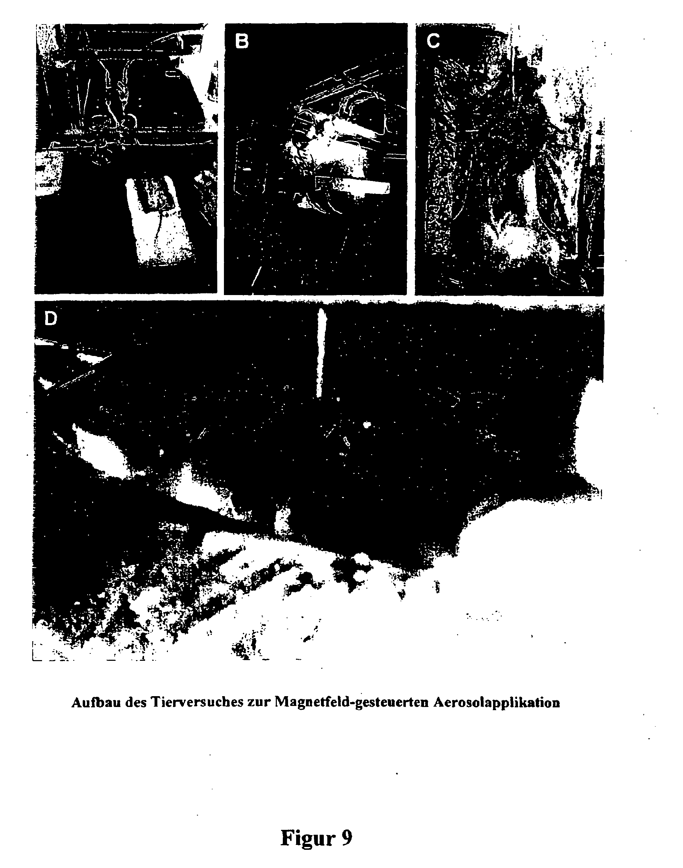

Erfindungsgemäß wurde gezeigt (siehe auch Figur 9), dass, die Deposition von Magnetpartikel durch die erfindungsgemäße Magnetfeld-geteuerte Deposition gezielt in Regionen bewirkt und auch erhöht werden kann, in denen normalerweise nur eine geringe Deposition erfolgt, wie bspw. oben für die trachebronchiale und die alveolare Region beschrieben.

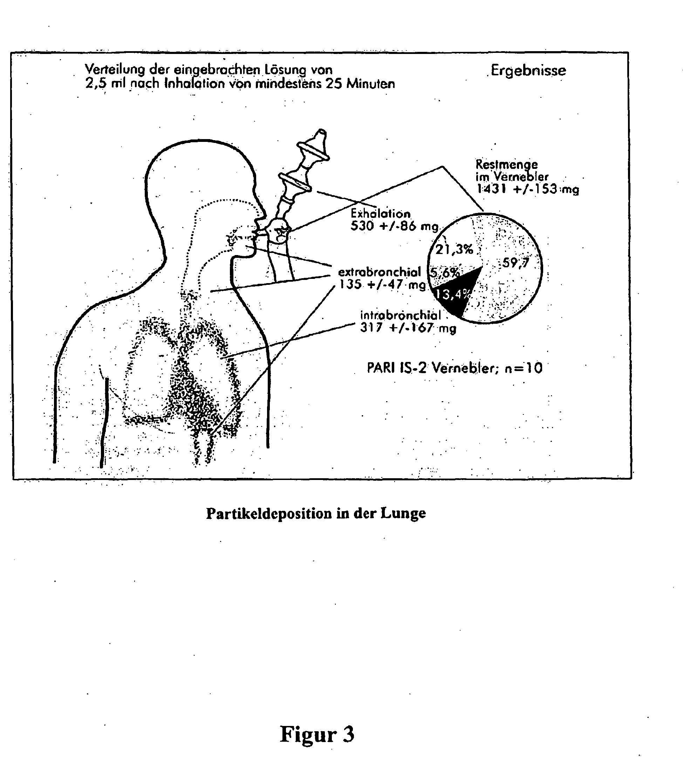

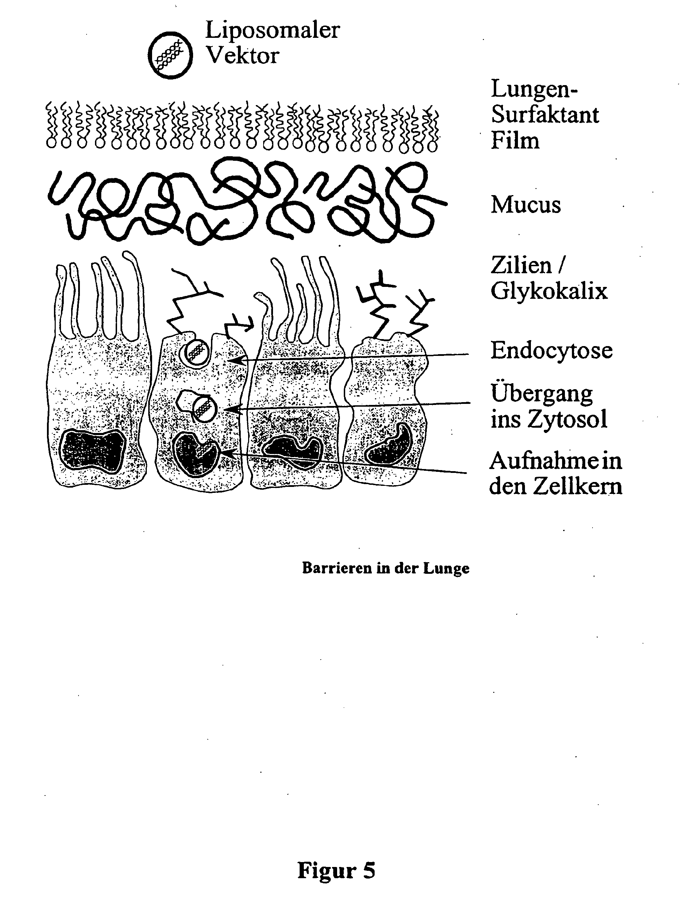

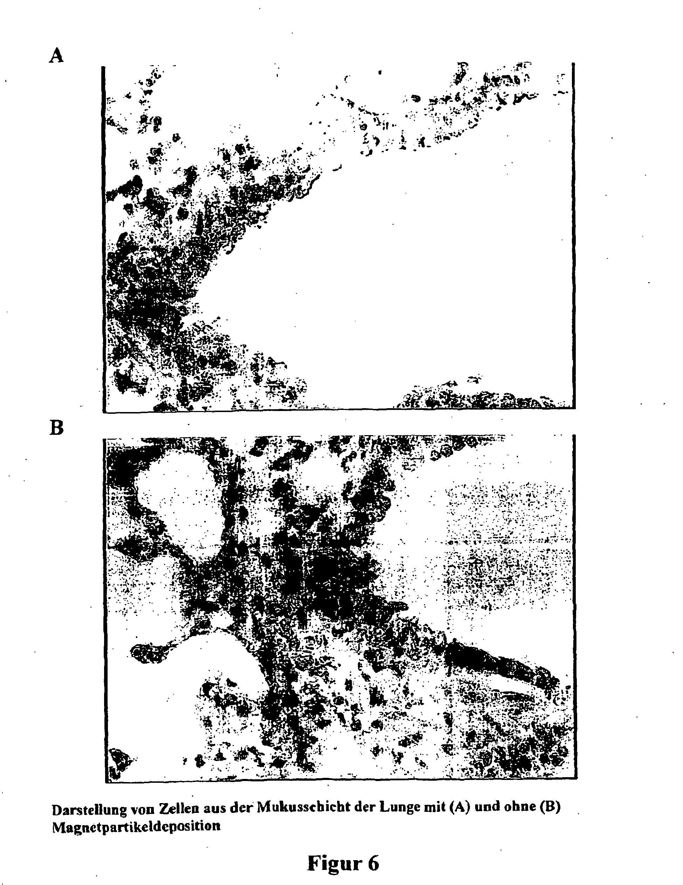

**Figur 3**    zeigt die Partikeldeposition bei Anwendung eines modernen pressluftbetriebenen Düsenverneblers (PARI IS-2). Von einer insgesamt inhalierten Aerosoldosis (Exhalation, extrabronchial und intrabronchial) werden 52 % wieder ausgeatmet (siehe Köhler, D. & Fischer, F. Theorie und Praxis der Inhalationstherapie (Arcis Verlag GmbH, München, 2000)). Diese Ergebnisse verdeutlichen, dass ein großer Anteil der inhalierten Aerosoldosis wieder exhaliert wird. Dieser exhalierte Anteil wird mit der vorliegenden Erfindung durch die Magnetfeld-gesteuerte Aerosoldeposition erheblich minimiert. Aus Figur 3 geht hervor, dass insgesamt nur 33 % der inhalierten Dosis in der Lunge deponiert. Die Ergebnisse aus Experimenten der Erfindung (dargestellt in den Figuren 5 und 6) zeigen, dass eine ca. 2,2- bis 2,5-fach Erhöhung dieser inhalierten Dosis deponiert wird, wenn der erfindungsgemäß Einfluss eines externen Magnetfeldes vorhanden ist. Da bei den vorgenannten Experimenten die Atembedingungen mit und ohne Magnetfeld identisch waren, resultiert, bei Übertragung der Ergebnisse auf die Daten aus Figur 3, daraus, dass sich mit den erfindungsgemäßen Aerosolen die in der Lunge deponierte Aerosoldosis von 33% auf ca. 82,5% steigern lässt.

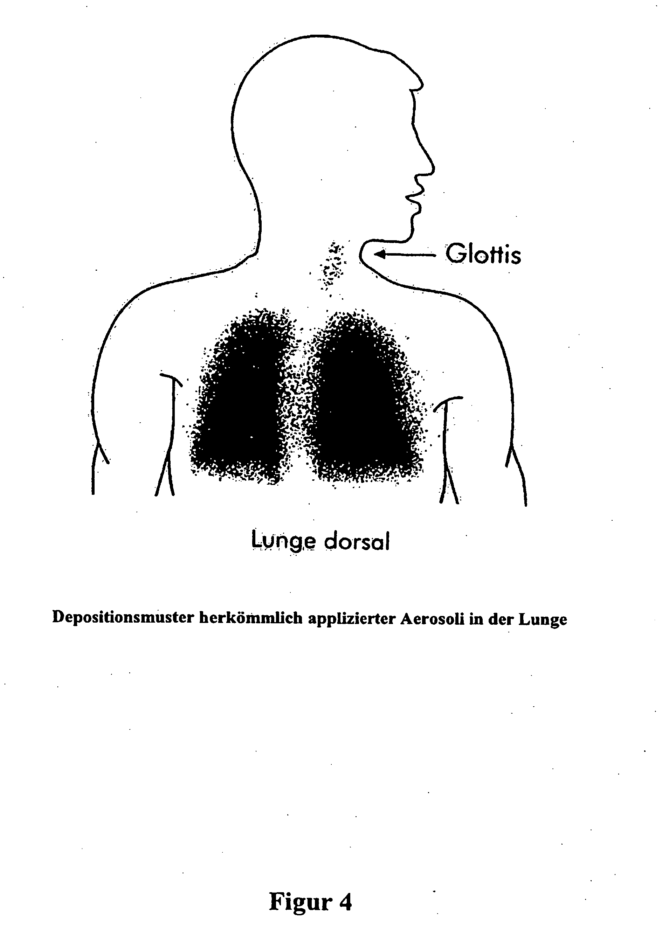

**Figur 4** zeigt das Depositionsmuster herkömmlich applizierter Aerosole bei Verwendung von Inhalationsmethoden, die dem Stand der Technik entsprechen. Wie zu erkennen ist, wird das inhalierte Aerosol homogen in der gesamten Lunge verteilt. Eine gerichtete Steuerung des Aerosols in definierte Regionen der Lunge ist mit den bisher bekannten Methoden nicht möglich.

**Figur 5** zeigt einen schematischen Querschnitt durch die Mukus-Schicht und das respiratorische Epithel. Diese Abbildung gibt einen Überblick über Bedingungen des *in vivo*-Transfers von pharmazeutischen Wirkstoffen (und Magnetpartikeln) der vorliegenden Erfindung. Nach topischer Applikation von beispielweise Genvektoren über die Atemwege sind extrazelluläre und intrazelluläre Barrieren zu überwinden, bis die DNA in den Zellkern gelangt. Der Lungensurfactantfilm, Mukus-Schicht, darin enthaltene Proteine, wie z.B. Nukleasen (DNase), bilden dabei eine erste Barriere. Weitere physikalische Barrieren sind Cilien und Glykokalix der Zelloberfläche. Nach intrazellulärer Aufnahme von Genvektoren muss die DNA vor dem lysosomalen Abbau geschützt und zum Zellkern transportiert werden. Die Zellkernporen der Kernmembran sind ein weiteres Hindernis für den erfolgreichen Transport. Die Mukus-Schicht ist *per se* eine schwer zu durchdringende Barriere. So kommt die Diffusion von Partikeln >500 nm durch die Mukus-Schicht nahezu zum erliegen (siehe Sanders, N. N., De Smedt, S. C. & Demeester, J. The physical properties of biogels and their permeability for macromolecular drugs and colloidal drug carriers [Review]. Journal of Pharmaceutical Sciences 89, 835-849 (2000)).

**Figur 6** stellt die unter der Mukus-Schicht liegenden Zellen der Lunge dar (wie in dem Querschnitt aus Figur 5 dargestellt). Figur 6A zeigt die Zellen nach Verabreichung eines erfindungsgemäßen Magnetpartikel-haltigen Aerosols unter Einfluss eines Magnetfeldes, Figur 6B zeigt die Zellen nach Verabreichung des entsprechenden erfindungsgemäßen Magnetpartikel- haltigen Aerosols ohne Einfluss eines Magnetfeldes. Wie in Figur 6A zu erkennen ist, adhärieren die Magnetparikel an der Zelloberfläche, sie sind als schwarze Ablagerungen zu erkennen. Dagegen sind in Figur 6B keine Ablagerungen an der Zelloberfläche detektierbar. Mit diesem Versuchsergebnis konnte gezeigt werden, dass die erfindungsgemäßen Magnetpartikel-haltigen Aerosole bzw. die enthaltenen Magnetpartikel und pharmazeutischen Wirkstoffe (die entweder an die Magnetpartikel gebunden sind oder mit diesen in einem Flüssigkeitstropfen enthalten sind, wie oben beschrieben) gerichtet durch die Mukus-Schicht transportiert werden, wohingegen dies ohne das erfindungsgemäße Anlegen eines externen Magnetfeldes nicht der Fall ist.

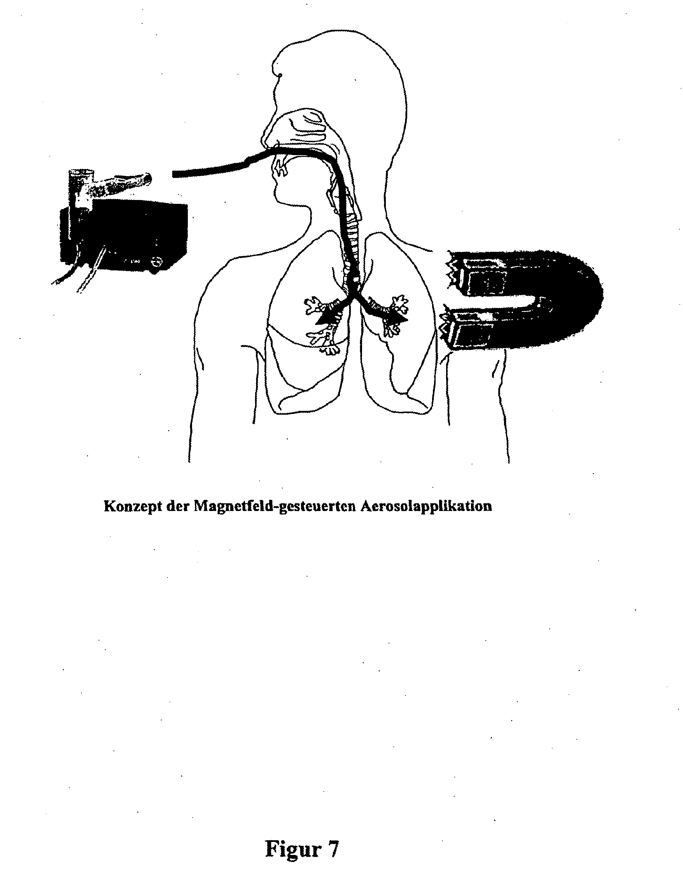

**Figur 7** zeigt eine schematische Darstellung des erfindungsgemäßen Konzepts der Magnetfeld-gesteuerten Aerosolapplikation. Ein erfindungsgemäßes Magnetpartikel-haltiges Aerosol wird mit einem kommerziell erhältlichen Vernebler (Fa. PARI, Starnberg, Deutschland) erzeugt. Die dabei entstehenden Magnetpartikel-haltigen Flüssigkeitstropfen werden von dem Patienten inhaliert und durch das extern angelegte Magnetfeld direkt in die gewünschten Bereiche der Lunge des Patienten gesteuert.

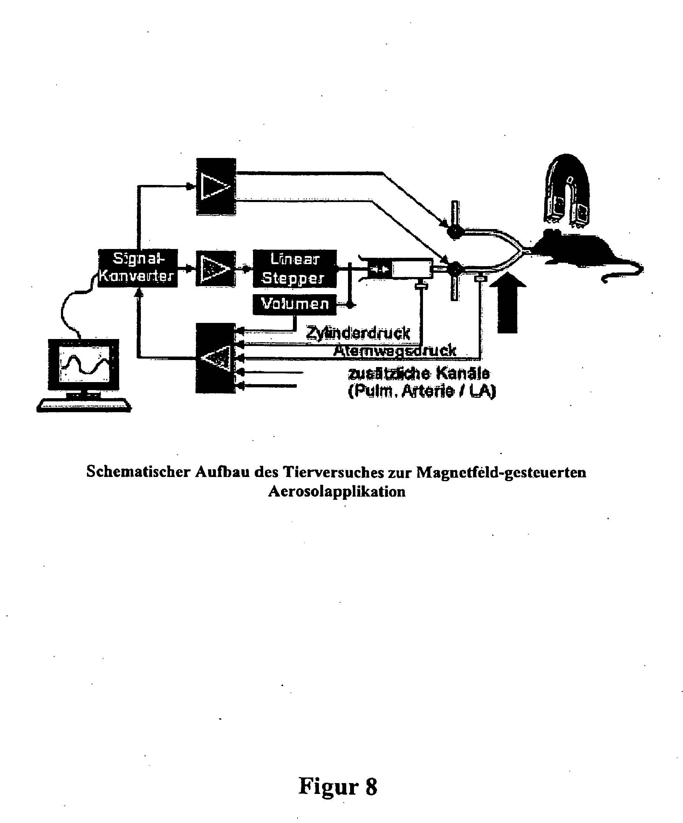

**Figur 8** zeigt eine schematische Darstellung eines Tierversuchs an der Maus des erfindungsgemäßen Konzepts der Magnetfeld-gesteuerten Aerosolapplikation. Magnetpartikel-haltige Aerosole wurden unter Einfluss eines angelegten Magnetfeldes gezielt in definierte Bereiche der Lunge einer Maus gesteuert.

**Figur 9** zeigt den Versuchsaufbau eines Tierversuchs an der Maus des erfindungsgemäßen Konzepts der Magnetfeld-gesteuerten Aerosolapplikation. In dem Mausmodell wurde der Brustkorb der Mäuse eröffnet, so dass einerseits die Trachea freipräpariert und intubiert werden konnte und andrerseits ein externes Magnetfeld an einer definierten Region der Lunge angelegt werden konnte. Die Maus wurde während der Aerosolapplikation beatmet (dargestellt ist das FlexiVent System der Firma SCIREQ) und das Aerosol durch einen angeschlossenen Vernebler in das Beatmungssystem eingeleitet (symbolisiert durch den roten Pfeil). Der Vernebler wurde mit der Beatmungsfrequenz synchronisiert, so dass nur während der Inspiration Aerosol in die Mauslunge appliziert wurde.
Der allgemeine Versuchsaufbau der intubierten Maus, die an das Beatmungsgerät angeschlossen ist, sowie der Vernebler ist in Figur 9A dargestellt. In Figur 9B ist die intubierte Maus fixiert unter dem Elektromagneten, der als externes Magnetfeld verwendet wurde, dargestellt. Die intubierte Maus mit geöffnetem Brustkorb (ohne den Magneten) ist in Figur 9C zuerkennen. In Figur 9D ist die auf der rechten Mauslunge platzierte Eisenspitze (Polschuh) des Elektromagneten gezeigt. Die Maus war während der Aerosolapplikation unter dem Elektromagneten fixiert, so dass die Eisenspitze des Elektromagneten nicht mit der linken Mauslunge in Kontakt kam. Der genaue Versuchsablauf wird in Beispiel 1 offenbart.

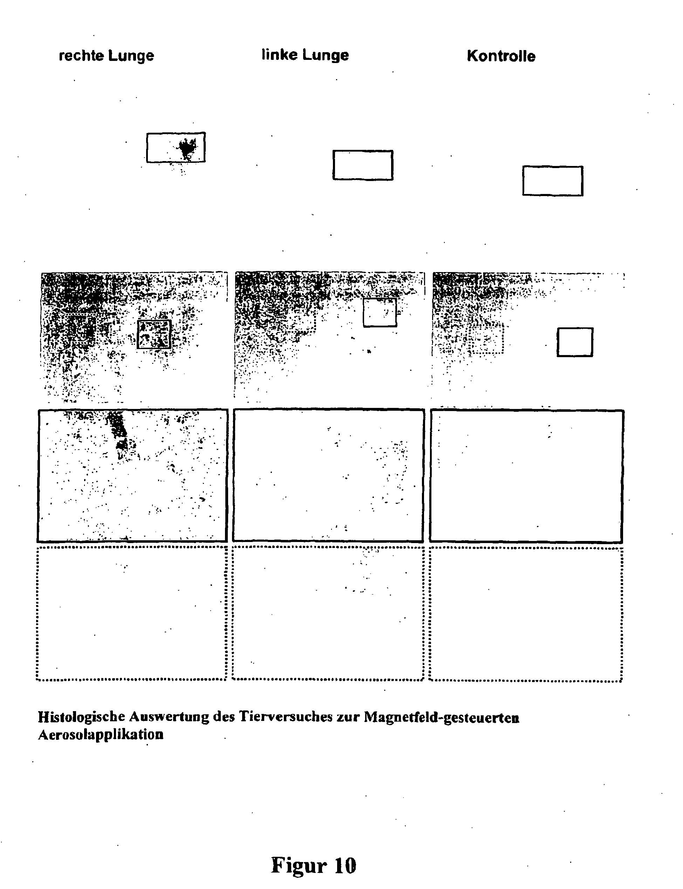

**Figur 10** stellt die histologische Auswertung eines Tierversuchs an der Maus der erfindungsgemäßen Magnetfeld-gesteuerten Aerosolapplikation dar. Es ist die Verteilung von superparamagnetischen Eisenoxidnanopartikel nach einer Aerosolapplikation in das Lungengewebe einer Maus dargestellt. Während der Aerosolapplikation wurde auf der rechten Lunge ein Magnetfeld angelegt. Die superparamagnetischen Eisenoxidnanopartikel erscheinen als Braunfärbung und sind in Figur 10 als dunkle Bereiche in der Abbildung. zu erkennen

In der ersten Reihe der Figur 10 sind Längsschnitte durch die Mauslunge dargestellt. Es ist deutlich die punktuelle Anreicherung der superparamagnetischen Eisenoxidnanopartikel an der Stelle des Polschuhs des Elektromagneten zu erkennen (linke Abbildung). Im Gegensatz dazu ist in der linken Lunge keine punktuelle Anreicherung der superparamagnetischen Eisenoxidnanopartikel, sondern eine homogene und diffuse Verteilung der superparamagnetischen Eisenoxidnanopartikel über die Lunge zu erkennen (mittlere Abbildung). Auch gezeigt ist eine Kontrolllunge, bei der erwartungsgemäß keine Braunfärbung der Lunge zu beobachten ist (rechte Abbildung). Bei allen Kontrolllungen der Figur 10 wurden keine superparamagnetischen Eisenoxidnanopartikel als Aerosol appliziert.

Die zweite Reihe der Figur 10 zeigt markierte Ausschnitte aus den Lungenschnitten der ersten Reihe als vergrößerte Darstellungen. Deutlich ist die Magnetfeld-induzierte Anreicherung der superparamagnetischen Eisenoxidnanopartikel in der rechten Mauslunge zu erkennen (linke Abbildung), während dieses nicht in der linken Mauslunge beobachtet wird (mittlere Abbildung). Ebenfalls sind Kontrolllungen dargestellt, in denen erwartungsgemäß keine Anreicherung von superparamagnetischen Eisenoxidnanopartikeln zu beobachten ist (rechte Abbildungen).

In der dritten und vierten Reihe der Figur 10 sind wiederum markierte Ausschnitte aus den Vergrößerungen der zweiten Reihe dargestellt. Eine deutliche Anreicherung der superparamagnetischen Eisenoxidnanopartikel in der Lunge ist nur in den Bereichen zu erkennen, in denen der Polschuh des Elektromagneten platziert wurde (linke Abbildungen). Ebenfalls sind Kontrolllungen dargestellt, in denen erwartungsgemäß keine Anreicherung von superparamagnetischen Eisenoxidnanopartikeln zu beobachten ist (rechte Abbildungen).

Diese in Figur 10 dargestellten Ergebnisse verdeutlichen, dass es möglich ist, ein erfindungsgemäßes Magnetpartikel-haltiges Aerosol erfolgreich mittels extern angelegter Magnetfelder kontrolliert in definierte Bereiche der Lunge zu steuern. Die Erfindung stellt damit einen wesentlichen Schritt in die Richtung einer völlig neuen Technologie zur Aersolapplikation und zur gezielten Therapie von zahlreichen Atemwegs- und/oder Lungenerkrankungen dar.

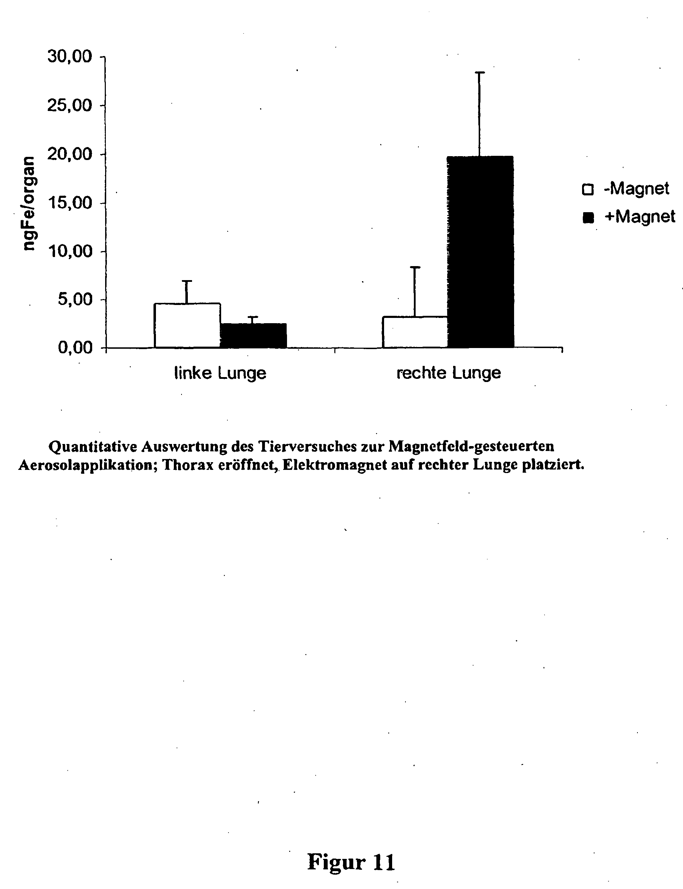

**Figur 11** stellt die quantitative Auswertung eines Tierversuchs an der Maus der erfindungsgemäßen Magnetfeld-gesteuerten Aerosolapplikation dar. Bei diesem Versuch war der Thorax der Maus eröffnet und ein Elektromagnet auf der rechten Lunge platziert. In Figur 11 werden die Ergebnisse der linken Lunge (ohne angelegtes Magnetfeld) denen der rechten Lunge (mit angelegtem Magnetfeld) gegenübergestellt.

In **Figur 11** ist die Menge der in den Lungen der Mäuse deponierten Menge von superparamagnetischen Eisenoxidnanopartikel unter Einfluss eines nur an der rechten Mauslunge angelegten Magnetfeldes dargestellt. Die Aerosolapplikation superparamagnetischer Eisenoxidnanopartikel in Abwesenheit eines Magnetfeldes führt zu einer gleichmäßigen Verteilung zwischen der rechten und linken Mauslunge (weiße Säulen). Im Gegensatz dazu bedingt das Anlegen eines Magnetfeldes an der rechten Mauslunge eine ca. 8-fache Anreicherung gegenüber der linken Mauslunge (schwarze Säulen).

Insgesamt betrachtet, ist die Gesamtdeposition superparamagnetischer Eisenoxidnanopartikel in der Mauslunge unter Einfluss des Magnetfeldes um mindestens das 2,8-fache höher als in Abwesenheit des Magnetfeldes. Auch durch diese Untersuchungen und Ergebnisse wurde erfindungsgemäß die gerichtete Deposition Magnetpartikel-haltiger Aerosole der vorliegenden Erfindung nachgewiesen.

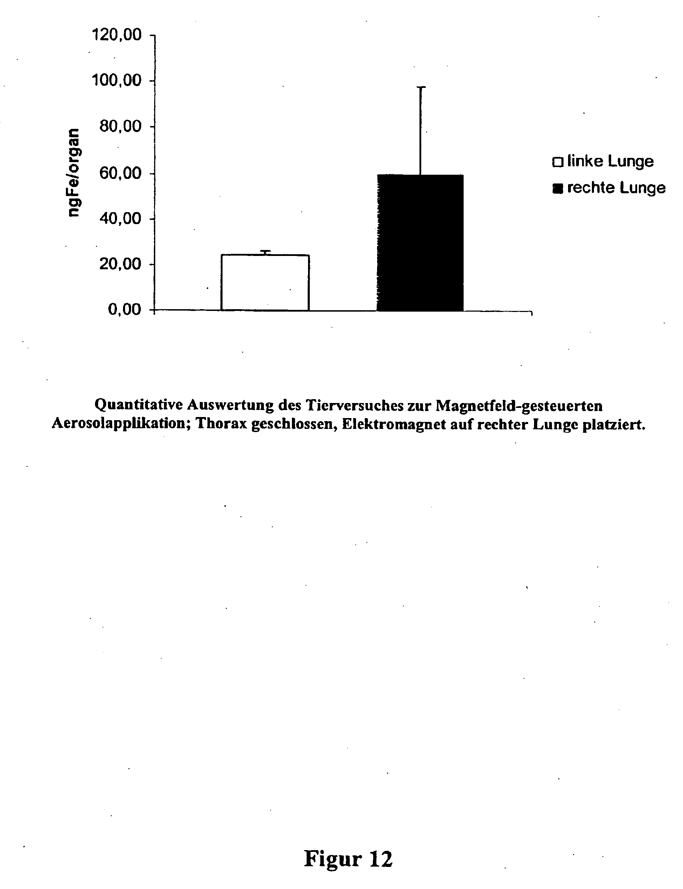

**Figur 12** stellt die quantitative Auswertung eines Tierversuchs an der Maus der erfindungsgemäßen Magnetfeld-gesteuerten Aerosolapplikation dar. Bei diesem Versuch war der Thorax der Maus geschlossen und ein Elektromagnet auf der rechten Lunge platziert. Figur 12 stellt die Ergebnisse der linken Lunge (ohne angelegtes Magnetfeld) denen der rechten Lunge (mit angelegtem Magnetfeld) gegenüber.

Hintergrund dieser Untersuchung war auch die humane Anwendung, bei der die Magnetfeld-gesteuerte Aerosolapplikation mit geschlossenem Thorax zu erfolgen hat. Wie in Figur 12 zu erkennen ist, resultiert auch bei geschlossenem Thorax der Einfluss des an der rechten Lunge angelegten Magnetfeldes in einer mindestens 2,5-fachen Erhöhung der Deposition der superparamagnetischen Eisenoxidnanopartikel und entspricht damit nahezu den Ergebnissen der Aerosolapplikation bei geöffnetem Thorax (dargestellt in Figur 11). Damit wurde nachgewiesen, dass die vorliegende Erfindung in der humanen Aerosoltherapie und unter den damit verbundenen Konditionen (geschlossener Thorax) anwendbar ist.

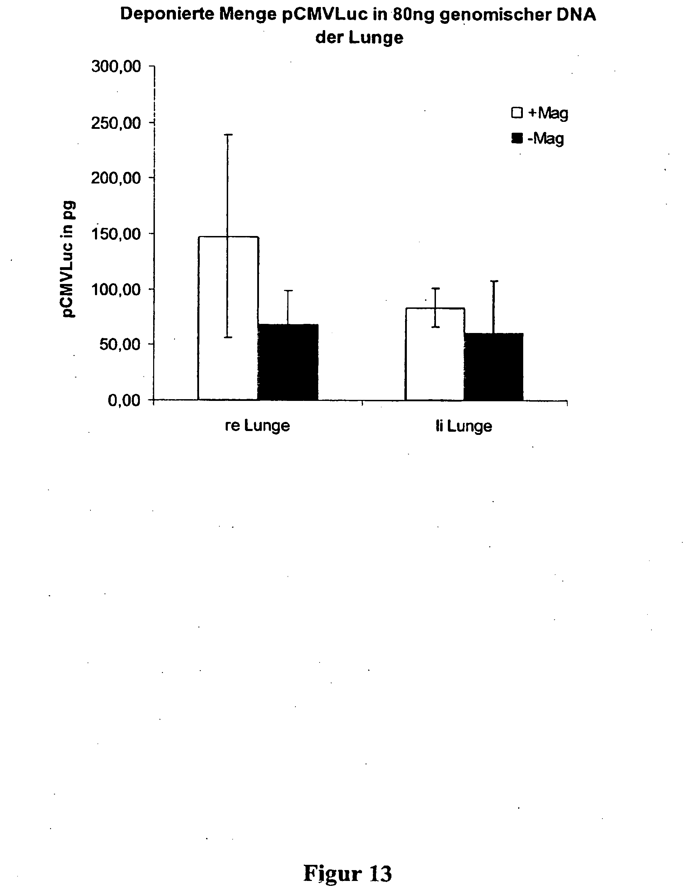

**Figur 13** stellt eine weitere quantitative Auswertung eines Tierversuchs an der Maus der erfindungsgemäßen Magnetfeld-gesteuerten Aerosolapplikation dar. Bei diesem Versuch wurde Plasmid-DNA (pCMVLuc) (als Wirkstoff) mit superparamagnetischen Eisenoxidnanopartikel formuliert und als erfindungsgemäßes Aerosol appliziert. Auch bei diesem Versuch war der Thorax der Maus geschlossen und ein Elektromagnet auf der rechten Lunge platziert. Figur 13 stellt die Ergebnisse der linken Lunge (ohne angelegtes Magnetfeld) denen der rechten Lunge (mit angelegtem Magnetfeld) gegenüber.

Hintergrund dieser Untersuchung war auch hier die humane Anwendung, bei der die Magnetfeld-gesteuerte Aerosolapplikation mit geschlossenem Thorax zu erfolgen hat. Wie aus Figur 13 zu entnehmen ist, resultiert bei geschlossenem Thorax der Einfluss des an der rechten Lunge angelegten Magnetfeldes in einer mindestens 2,2-fachen Erhöhung der Deposition der applizierten, mit superparamagnetischen Eisenoxidnanopartikel formulierten, Plasmid-DNA und entspricht damit nahezu den Ergebnissen der Aerosolapplikation bei geöffnetem Thorax (dargestellt in Figur 10).

Damit wurde abermals nachgewiesen, dass die vorliegende Erfindung in der humanen Aerosoltherapie und unter den damit verbundenen Konditionen (geschlossener Thorax) anwendbar ist.

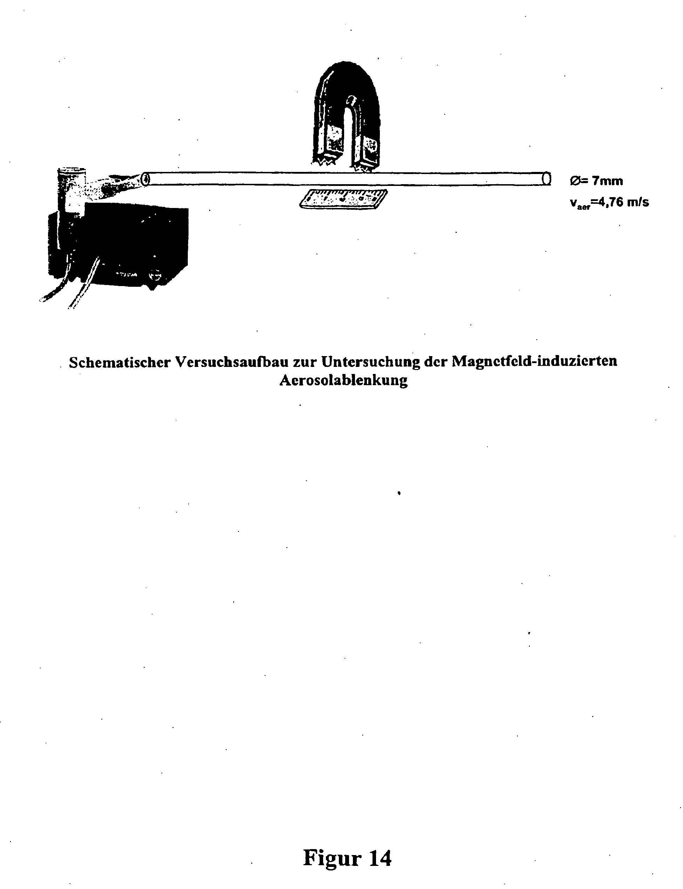

**Figur 14** stellt einen schematischen Versuchsaufbau zur Untersuchung der Magnetfeld-induzierten Aerosolablenkung dar.

Für den erfindungsgemäßen Magnetfeld-gesteuerten pharmazeutischen Wirkstofftransfer in der Aerosoltherapie wurde ein Testmodell etabliert. Der Versuchsaufbau des Testmodells ist schematisch in Figur 14 dargestellt. Das Testmodell ist durch folgende Merkmale gekennzeichnet: Ein Standardvernebler (PARI LC plus; zur Verfügung gestellt durch die Partnerfirma PARI GmbH, Starnberg) wurde an einen 60 cm langen Kunststoffschlauch mit einem Innendurchmesser von 0,7 cm angeschlossen und verschiedene Magneten in einem Abstand von 30 cm von dem Vernebler an der äußeren Schlauchwand positioniert. Die gewählten Vernebelungsbedingungen entsprechen dem Durchmesser und der Luftgeschwindigkeit wie sie in der 1. - 2. Bronchialverzweigung der humanen Lunge vorzufinden sind. Superparamagnetische Eisenoxidnanopartikel-haltige wässrige erfindungsgemäße Formulierung wurden für definierte Zeiten in den Schlauch vernebelt und die Magnetfeld-abhängige Ablenkungseffizienz mittels des Vermessens der Eisenoxidnanopartikel-haltigen Schlauchfläche quantifiziert.

**Figur 15** zeigt die Magnetfeld-induzierte Aerosolablenkung superparamagnetische Eisenoxidpartikel-haltiger Aerosole.

Zur Veranschaulichung der Magnetfeld-induzierten Ablenkung erfindungsgemäßer Eisenoxidpartikel-haltiger Aerosole, ist in Figur 15 die Abscheidung der Eisenoxidpartikel an der Innenwand des Schlauches des in Figur 14 dargestellten Testmodells exemplarisch gezeigt. Bei diesem Versuch wurde ein Stabmagnet mit einer aufgesetzten Eisenspitze verwendet. Die Verneblungsparameter sind angegeben. Auf dem oberen Foto der Figur 15 ist die Aerosoldeposition im Schlauch ohne Applikation des Magnetfeldes als Kontrolle gezeigt. Es ist deutlich zu erkennen, dass die Eisenoxidpartikel homogen auf dem Boden des Schlauches deponieren, wenn kein Magnetfeld angelegt wird. Im Gegensatz dazu ist deutlich die Anreicherung der Eisenoxidpartikel an der Eisenspitze des Permanentmagneten bei Applikation des Magnetfeldes zu erkennen. Ebenfalls ist deutlich zu erkennen, dass sich die Flugbahn der Aerosolpartikel in Abhängigkeit des Magnetfeldes ändert, da keine Deposition auf dem Boden des Schlauches unterhalb der Eisenspitze des Magneten der Eisenpartikel zu erkennen ist.

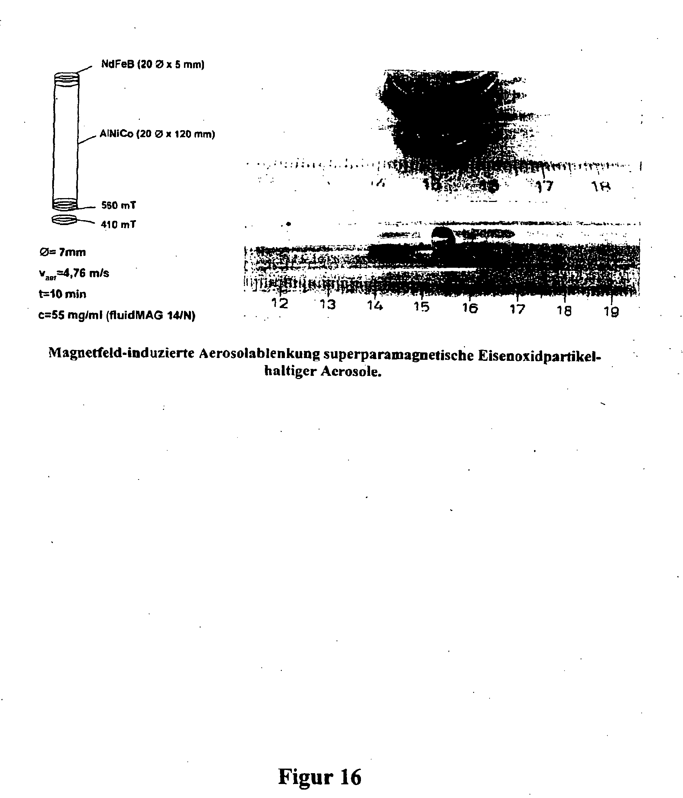

**Figur 16** zeigt die Magnetfeld-induzierte Aerosolablenkung erfindungsgemäßer superparamagnetische Eisenoxidpartikel-haltiger Aerosole.

Mittels des in Figur 14 dargestellten Testmodels wurden verschiedene Magnetanordnungen untersucht. Es stellte sich vor allem heraus, dass die Erzeugung von Magnetfeldgradienten, durch das gezielte Platzieren von Eisenstücken (z.B. Büroklammern) in dem Magnetfeld, die Abscheidung der Aerosole stark erhöhte. Die effizienteste Anordnung wurde durch die Platzierung eines Permanentmagneten jeweils ober- und unterhalb des Schlauches erreicht. Das Resultat ist in Figur 16 gezeigt.

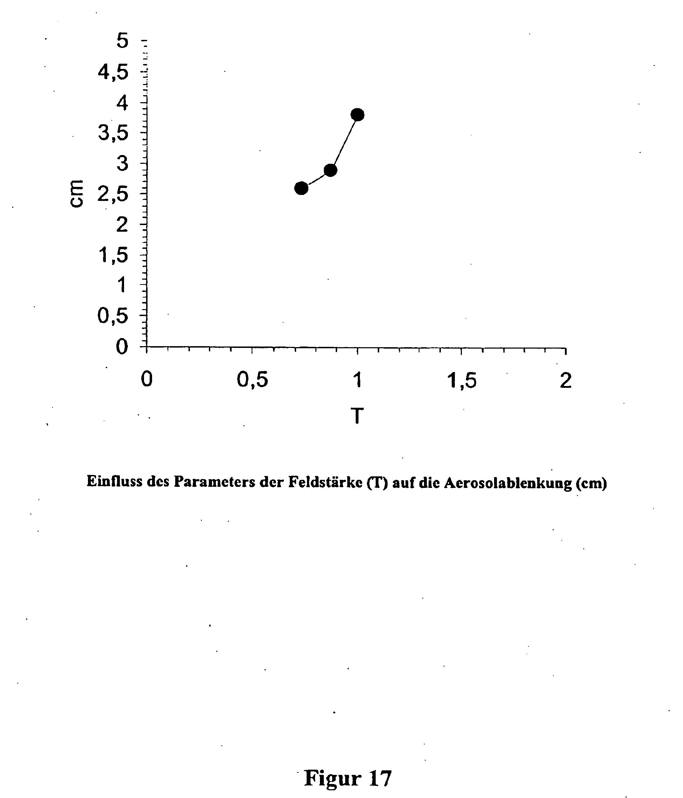

**Figur 17** zeigt den weiteren Parameter der Feldstärke, der einen Einfluss auf das Ablenkungsverhalten erfindungsgemäßer superparamagnetischer Eisenoxidpartikel-haltigen Aerosole hat. Die Untersuchungen erfolgten anhand des in Figur 14 dargestellten Testmodells.

Es ist deutlich zu erkennen, dass die Abscheidung der superparamagnetischen Eisenoxidpartikel-haltigen Aerosole stark mit der Magnetfeldstärke abnimmt. Wie erwartet gilt, je größer das Magnetfeld ist, desto höher ist die Ablenkung des Aerosols.

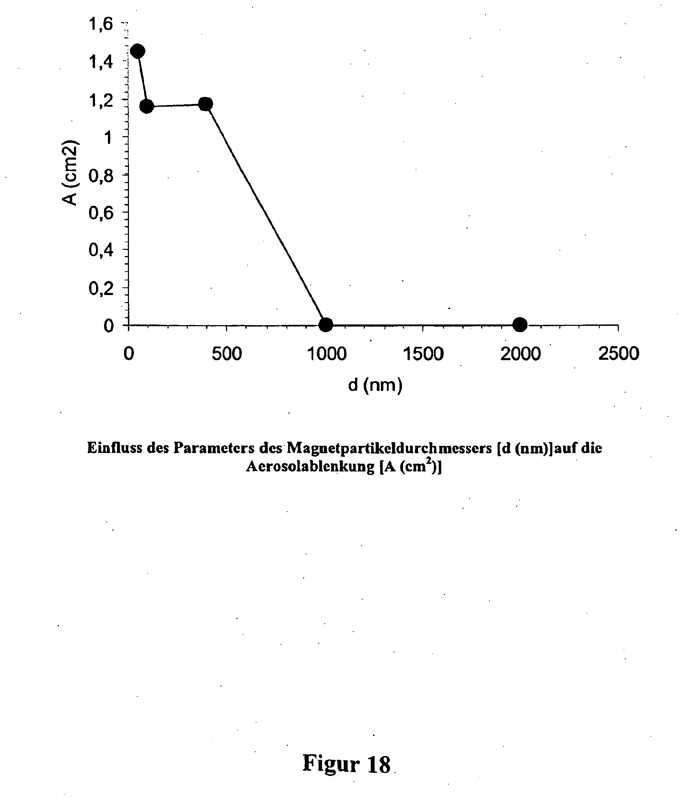

Figur 18 zeigt den weiteren Parameter des Magnetpartikeldurchmessers, der einen Einfluss auf das Ablenkungsverhalten erfindungsgemäßer superparamagnetische Eisenoxidpartikel-haltigen Aerosole hat. Die Untersuchungen erfolgten anhand des in Figur 14 dargestellten Testmodells untersucht.

Es ist zu erkennen, dass die Abscheidung des Aerosols abhängig ist von der Größe des Durchmessers der superparamagnetischen Eisenoxidpartikel. Interessanterweise ist die Ablenkung kleiner Partikel mit 50 nm Durchmesser durchaus effektiver als die größerer Partikel von über 800 nm bei gleicher Eisenkonzentration. Die erfindungsgemäß verwendeten Magnetpartikel mit einem Durchmesser von bis zu 800 Nanometer zeigen daher deutlich bessere Ergebnisse für den Aerosoltransport als Magnetpartikel mit einem größeren Durchmesser als 800 Nanometer. Somit sind die erfindungsgemäßen Magnetpartikel-haltigen Aerosole ausgezeichnet für die Anwendung in der Aerosoltherapie geeignet.

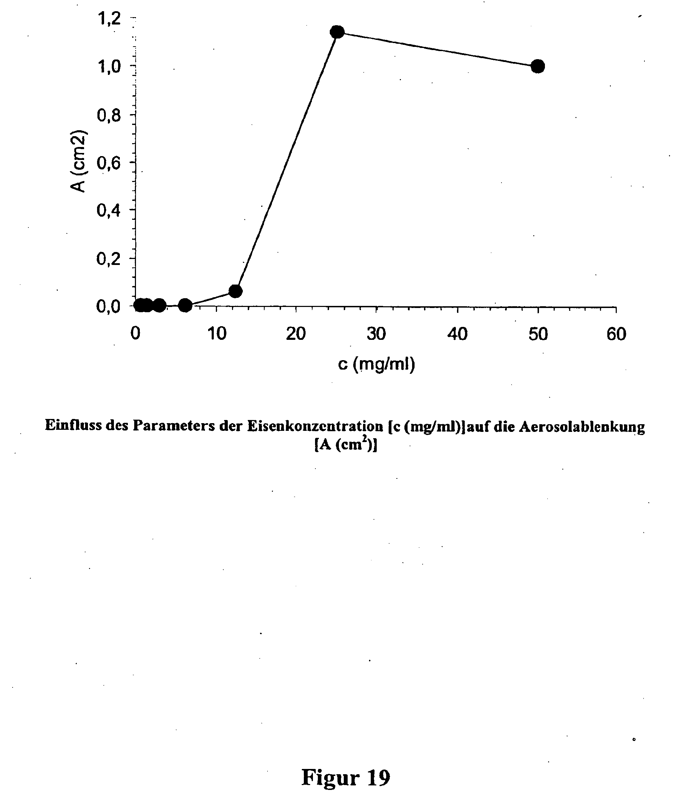

**Figur 19** zeigt den weiteren Parameter der Eisenkonzentration, die einen Einfluss auf das Ablenkungsverhalten erfindungsgemäßer superparamagnetische Eisenoxidpartikel-haltigen Aerosole hat. Die Untersuchungen erfolgten anhand des in Figur 14 dargestellten Testmodells.

Ein weiterer Parameter, der einen großen Einfluss auf das Ablenkungsverhalten hat, ist die Konzentration der vernebelten superparamagnetischen Eisenoxidpartikel in der Aerosollösung. In Figur 19 ist dieses exemplarisch für 100 nm große Partikel gezeigt. Es ist zuerkennen, dass eine Mindestkonzentration von 12,5 mg/ml Lösung verwendet werden muss, damit sich die superparamagnetischen Eisenoxidpartikel-haltigen Aerosole ablenken lassen.

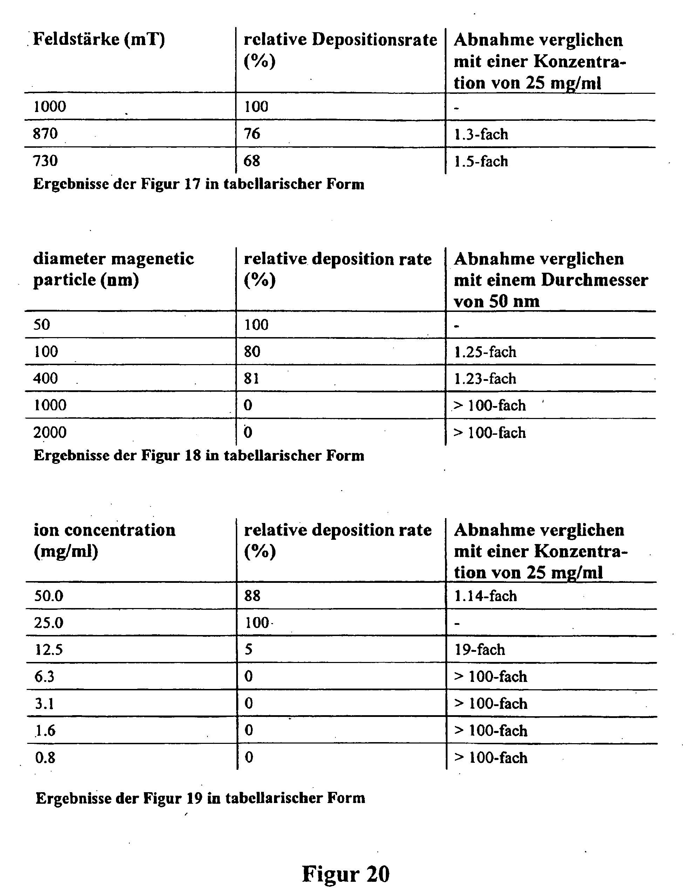

**Figur 20** zeigt in Tabellen die Werte der Figur 17, Parameter der Feldstärke; Figur 18, Parameter des Magnetpartikeldurchmessers; und Figur 19, Parameter der Eisenkonzentration.

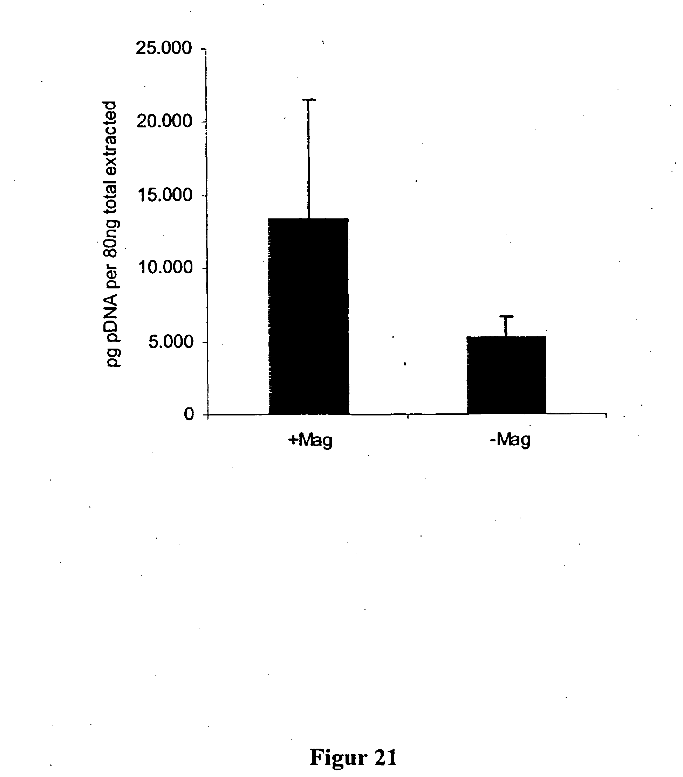

**Figur 21** beschreibt die Ergebnisse von *in vivo*-Versuchen mit BALB/c-Mäusen (siehe Beispiel 4). Dazu wurden BALB/c Mäusen auf dem Brustkorb zwei Permanentmagneten fixiert und ein erfindungsgemäßes Magnetpartikel- haltiges Aerosol verabreicht. 24 Stunden nach Applikation des erfindungsgemäßen Magnetpartikel-haltigen Aerosols wurden die Mäuse (n=3) getötet und die Menge an Plasmid DNA in den Lungen mittels Real Time PCR bestimmt. Wie in Figur 21 zu sehen, führte die Anwesenheit der Permanentmagneten auf dem Brustkorb zu einer 2,5-fach höheren Deposition von Plasmid DNA in der Lunge.

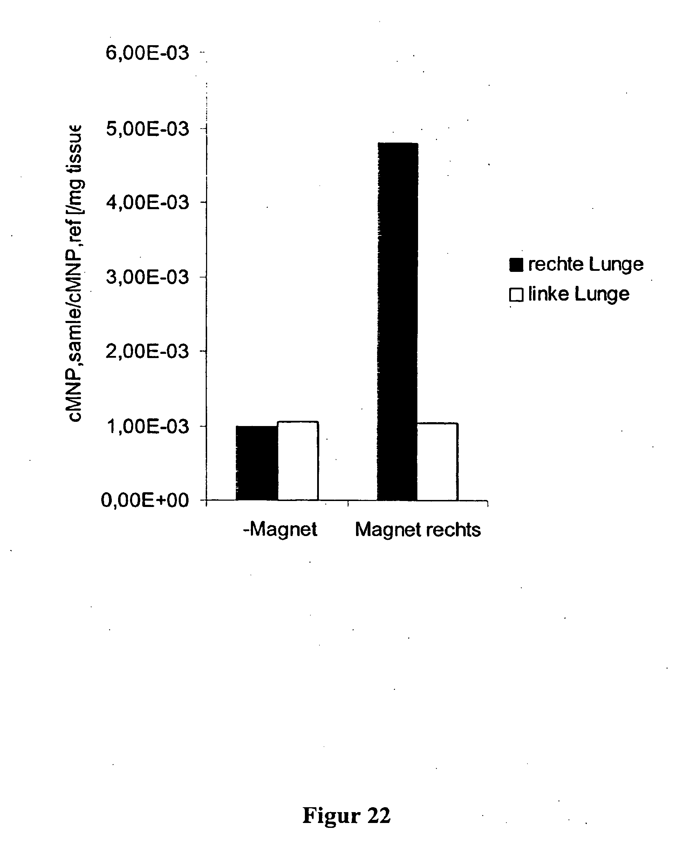

**Figur 22** beschreibt die Ergebnisse von *in vivo*-Versuchen mit narkotisierten Hausschweinen. (siehe Beispiel 5). Dazu wurden zwei BALB/c narkotisierten Hausschweinen ein erfindungsgemäßes Magnetpartikel- haltiges Aerosol verabreicht. Bei dem ersten Schwein wurde ein Permanentmagnet auf der rechten Brustseite und bei dem zweiten Schwein kein Magnet während der Applikation platziert. Im Anschluss an die Aerosolapplikation wurden die Schweine getötet und die Lungen entnommen. In den ventralen Lungenbreichen wurden die Magnetpartikel mittels Magnetrelaxometrie quantifiziert. Wie in Figur 22 zu sehen ist, die Anzahl Magnetpartikel in der rechten Lunge mittels Magnetfeld ist um das 5-fache höher als ohne Magnetfeld.

### Beispiele

### Beispiel 1: Verabreichung eines erfindungsgemäßen Magnet-partikel-haltigen Aerosols

BALB/c Mäuse wurden mit einer intraperitonealen Injektion Pentobarbital narkotisiert. Nach sistieren des Kornealreflexes wurden die Tiere intubiert bzw. über eine Tracheotomie an das Flexivent System mit Vernebler angeschlossen. Die Mäuse wurden mit einer Atemfrequenz von $f = 120$ min$^{-1}$ und einem Tidalvolumen von $TV = 10$ $\mu$l/g bei einem PEEP von 4 cmH$_2$O Volumenkontrolliert beatmet. Vor Beginn der Inhalation wurde eine Messung der Lungenimpedanz als Ausgangswert durchgeführt (Pertubation + snapshot).

Der Polschuh (Eisenspitze) eines Elektromagneten wurde über der rechten Mauslunge platziert und mit einer Stromstärke von $I = 10$ A (1 T) betrieben. 0,7 ml einer wässrigen erfindungsgemäßen. Formulierung mit superparamagnetischen Eisenoxid Nanopartikeln (fluidMAG-PEI 50 nm, c = 6,25 mg/ml) wurden in 10 s Intervallen (n = 20, output rate = 200 $\mu$l/min) appliziert. Am Ende des Experiments wurden die Mauslungen entnommen und die rechte und linke Mauslunge getrennt voneinander in flüssigem Stickstoff tief gefroren. Die Quantifizierung des Magnetpartikelgehaltes in den Lungen wurde mittels Magnetrelaxometrie durchgeführt (Lange J, et al., Magnetorelaxometry, a new binding specific detection method based on magnetic nanoparticles, JOURNAL OF MAGNETISM AND MAGNETIC MATERIALS 252 (1-3): 381-383 NOV 2002). Die Versuchsgruppengröße umfasste 4 Mäuse (3 Mäuse zur Quantifizierung des Magnetpartikelgehaltes der Lungen mittels Magnetrelaxometrie und eine Maus für die histologische Aufarbeitung (Kryoschnitte)).

**Beispiel 2: Magnet-partikel-haltiges Aerosol mit Plasmid-DNA als pharmazeutischem Wirkstoff**

Superparamagnetische Eisenoxidnanopartikel, die mit dem kationischen Polymer Polyethylenimin beschichtet sind (50 nm) (fluidMAG-PEI, Chemicell, Berlin, Deutschland) werden in destilliertem Wasser auf eine Eisenendkonzentration von 12,5 mg/ml verdünnt. Das Volumen beträgt 4 ml. Plasmid-DNA (pCMVLuc) wird mit destilliertem Wasser auf eine Konzentration von 0,625 mg/ml verdünnt (Endvolumen 4 ml). Daraus resultiert eine Gesamtdosis von 5 mg DNA. Die Plasmid-DNA-Lösung wird zu der Eisenoxidnanopartikel-Lösung pipettiert und durch Auf- und Abpipettieren gut gemischt. Der resultierende pH-Wert der Lösung wird auf pH = 7.0 eingestellt. Die Lösung wird 10 Min. vor der Applikation durch Vernebelung bei Raumtemperatur inkubiert. Es ist darauf zu achten, dass die Elektrolyt-Konzentration möglichst niedrig gehalten wird, um eine Salz-induzierte Aggregation der Eisenoxidnanopartikel zu verhindern. Aus diesem Grund sollten zur Isotonisierung von Lösungen, keine Elektrolyte, sondern nichtionische Substanzen, wie bspw. Glucose oder Glycerol, verwendet werden. Die Verneblungsdauer ist abhängig von dem Verneblertyp und beträgt für einen PARI BOY (PARI GmbH, Starnberg, Deutschland) ca. 15 Min für 4 ml Volumen mit einer Ausstoßrate von 5 - 6 l/min. Daraus ergibt sich eine Vernebelungszeit von ca. 30 Min. Das angelegte Magnetfeld wird auf einen Magetfeldgradienten von mindestens 10 T/m, eingestellt und während der Vernebelung an der gewünschten Position der Lunge platziert.

**Beispiel 3: Magnet-partikel-haltiges Aerosol mit Zytostatika als pharmazeutischem Wirkstoff**

Doxycylin-HCl wird in 20%-iger ethanolischer wässriger Lösung mit einer Konzentration von 16 mg/ml in einem Volumen von 4 ml gelöst. Zu dieser Lösung werden 4 ml einer Lösung von superparamagnetischen Eisenoxidnanopartikel (c=12,5 mg/ml, 50 nm, fluidMag-PEI, Chemicell, Berlin, Deutschland) pipettiert. Die Lösung wird unter den in Beispiel 2 genannten Bedingungen vernebelt.

Paclitaxel wird in einer Mischung aus Ethanol und Polyethylenglycol200 (PEG-200) mit einer Konzentration von 75 mg/ml in einem Volumen von 4 ml gelöst. Zu dieser Lösung werden 4 ml einer Lösung von superparamagnetischen Eisenoxidnanopartikel (c=12,5 mg/ml, 50 nm, fluidMag-PEI, Chemicell, Berlin, Deutschland) pipettiert. Die Lösung wird unter den in Beispiel 2 genannten Bedingungen vernebelt.

Dilauroylphosphatidylcholin und Paclitaxel werden in einem Verhältnis von 10:1 (w/w) in t-Butanol gelöst, bei -70°C eingefroren und lyophilisiert. Bis-zum Gebrauch wird das Lyophilisat bei -20°C gelagert. Vor Gebrauch wird das Lyophilisat mit sterilem destillierten Wasser rekonstituiert und gevortext bis sich mulilamellare Liposomen bilden. Die Endkonzentration von Paclitaxel beträgt 10 mg/ml in 4 ml Gesamtvolumen. Zu dieser Lösung werden 4 ml einer Lösung von superparamagnetischen Eisenoxidnanopartikel (c=12,5 mg/ml, 50 nm, fluidMag-PEI, Chemicell, Berlin, Deutschland) pipettiert. Die Lösung wird unter den in Beispiel 2 genannten Bedingungen vernebelt.

**Beispiel 4: *In Vivo*-Versuche mit BALB/c-Mäusen**

BALB/c Mäusen (Elevage Janvier, Route Des Chenes Secs, Le Genest Saint Isle, 53940 Frankreich) wurden auf dem Brustkorb zwei Permanentmagneten (NeoFeBr, 10x10x10mm; 500 mT) mit Gewebekleber fixiert. Die Mäuse wurden in eine Plexiglasbox, die in sechs gleichgroße Kammern aus aerosoldurchlässigem Draht unterteilt war, gesetzt. In die einzelnen Kammern wurde abwechselnd eine Maus mit oder ohne Magnet platziert. Zur Herstellung des erfindungsgemäßen Magnetpartikel-haltigen Aerosols wurde Plasmid DNA, kodierend für das Luizerfasegen, mit destilliertem Wasser auf ein Volumen von 5 ml (c=0,2 mg/ml) verdünnt. Dann wurden Superparamagnetische Eisenoxid-Nanopartikel (fluidmag-PEI, 50 nm) mit destilliertem Wasser auf ein Volumen von 5 ml (c=3 mg/ml) verdünnt, die DNA Lösung schnell zu der Nanopartikellösung pipettiert und durch mehrmaliges Auf- und Abpipettieren gut gemischt. Im Anschluss, wurde die Lösung mittels eines Verneblers an die Mäuse in der Plaexiglasbox appliziert. Die Lösung wird unter den in Beispiel 2 genannten Bedingungen vernebelt. Das allgemeine Prozedere zur Aerosol-Applikation ist in der Publikation Rudolph *et al.* (2005) beschrieben (Rudolph C, Ortiz A, Schillinger U, jauernig J, Plank C, Rosenecker J. Methodological optimization of polyethylenimine (PEI)-based gene delivery to the lungs of mice via aerosol application. J Gene Med. 2005 Jan;7(1):59-66.; Rudolph C, Schillinger U, Ortiz A, Plank C, Golas MM, Sander B, Stark H, Rosenecker J., Aerosolized nanogram quantities of plasmid DNA mediate highly efficient gene delivery to mouse airway epithelium., Mol Ther. 2005 Sep;12(3):493-501.). 24 Stunden nach Applikation des erfindungsgemäßen Magnetpartikel-haltigen Aerosols wurden die Mäuse (n=3) getötet und die Menge an Plasmid DNA in den Lungen mittels Real Time PCR bestimmt. Die Anwesenheit der Permanentmagneten auf dem Brustkorb führte zu einer 2,5-fach höheren Deposition von Plasmid DNA in der Lunge (vgl. Figur 21). Diese Ergebnisse zeigen, dass auch Permanentmagneten dazu geeignet sind Arzneistoffe in Magnetpartikel-haltigen Aerosolen in der Lunge *in vivo* anzureichern:

**Beispiel 5: *In Vivo*-Versuche mit narkotisierten Hausschweinen**

Zwei narkotisierte Hausschweine (ca. 20 kg) wurden unter kontrollierten Bedingungen beatmet und während der Beatmung ein wie in den Beispielen 1, 2 und 3 beschriebenes erfindungsgemäßes Magnetpartikel-haltiges Aerosol appliziert (10 ml fluidMAG PEI, 50 nm, 25 mg/ml). Bei dem ersten Schwein wurde ein Permanentmagnet (4 übereinanderliegende 8x4x2 cm Magneten, 0,8 T) auf der rechten Brustseite und bei dem zweiten Schwein kein Magnet während der Applikation platziert. Im Anschluss an die Aerosolapplikation wurden die Schweine getötet und die Lungen entnommen. In den ventralen Lungenbreichen wurden die Magnetpartikel mittels Magnetrelaxometrie quantifiziert. Die Anzahl Magnetpartikel in der rechten Lunge mittels Magnetfeld ist um das 5-fache höher als ohne Magnetfeld (siehe Figur 22). Diese Versuche zeigen, dass erfindungsgemäße Magnetpartikel-haltige Aerosole auch gezielt in die Lunge großer Versuchstiere gesteuert werden können.

**Patentansprüche**

1.  Magnetpartikel-haltiges Aerosol, wobei das Aerosol Magnetpartikel mit einem Durchmesser von mindestens 5 nm und höchstens 800 nm sowie mindestens einen pharmazeutischen Wirkstoff enthält.

2.  Magnetpartikel-haltiges Aerosol nach Anspruch 1, wobei die Magnetpartikel einen Durchmesser von mindestens 50 nm und höchstens 750 nm, bevorzugt von mindestens 100 nm und höchstens 700 nm, stärker bevorzugt von mindestens 150 nm und höchstens 600 nm, noch stärker bevorzugt von mindestens 200 nm und höchstens 500 nm, insbesondere bevorzugt von mindestens 250 nm und höchstens 450, am stärksten bevorzugt von mindestens 300 nm und höchstens 400 nm aufweisen.

3.  Magnetpartikel-haltiges Aerosol nach Anspruch 1 oder 2, wobei die Magnetpartikel und der pharmazeutische Wirkstoff in einem Lösungsmittel enthalten sind.

4.  Magnetpartikel-haltiges Aerosol nach Anspruch 3, wobei das Lösungsmittel ein anorganisches oder organisches Lösungsmittel ist.

5.  Magnetpartikel-haltiges Aerosol nach Anspruch 3 oder 4, wobei das Lösungsmittel ausgewählt ist aus der Gruppe bestehend aus Ethanol, Wasser und Glycerin sowie Mischungen hiervon.

6.  Magnetpartikel-haltiges Aerosol nach einem der Ansprüche 1 bis 5, wobei der pharmazeutische Wirkstoff an die Magnetpartikel gebunden ist.

7.  Magnetpartikel-haltiges Aerosol nach einem der Ansprüche 1 bis 5, wobei der pharmazeutische Wirkstoff an die Magnetpartikel nicht gebunden ist.

8.  Magnetpartikel-haltiges Aerosol nach einem der Ansprüche 1 bis 7, wobei der pharmazeutische Wirkstoff ausgewählt ist aus der Gruppe bestehend aus Nukleinsäuren, Peptiden, Proteinen, Zytostatika, Broncholytika, Antibiotika, Antidiabetika und Immunmodulatoren.

9.  Magnetpartikel-haltiges Aerosol nach einem der Ansprüche 1 bis 8, wobei der pharmazeutische Wirkstoff in einem Vektor, Liposom, Hohlkolloid oder Nanopartikel enthalten ist.

10. Magnetpartikel-haltiges Aerosol nach einem der Ansprüche 1 bis 9, wobei die Magnetpartikel aus Metallen und/oder deren Oxiden und/oder Hydroxiden bestehen oder diese enthalten.

11. Magnetpartikel-haltiges Aerosol nach einem der Ansprüche 1 bis 10, wobei die Magnetpartikel aus Metallen bestehen oder diese enthalten, ausgewählt aus der Gruppe, bestehend aus Eisen, Kobalt oder Nickel, magnetische Eisenoxide oder -hydroxide, wie $Fe_3O_4$, gamma-$Fe_2O_3$, Doppel-Oxide oder -Hydroxide aus zwei- oder dreiwertigen Eisenionen mit zwei- oder dreiwertigen anderen Metallionen, wie $Co^{2+}$, $Mn^{2+}$, $Cu^{2+}$, $Ni^{2+}$, $Cr^{3+}$, $Gd^{3+}$, $Dy^{3+}$ oder $Sm^{3+}$, sowie beliebige Mischungen hiervon.

12. Magnetpartikel-haltiges Aerosol nach einem der Ansprüche 1 bis 11, wobei die Magnetpartikel aus paramagnetischem oder superparamagnetischem Material bestehen oder dieses enthalten.

13. Pharmazeutische Zusammensetzung, enthaltend Magnetpartikel-haltiges Aerosol nach einem der Ansprüche 1 bis 12 sowie gegebenenfalls geeignete Hilfs- und/oder Zusatzstoffe.

14. Pharmazeutische Zusammensetzung nach Anspruch 13, zusätzlich enthaltend mindestens ein (weiteres) Lösungsmittel, mindestens einen Komplexbildner und/oder mindestens eine pharmazeutisch verträgliche Säure.

15. Magnetpartikel-haltiges Aerosol nach einem der Ansprüche 1 bis 12 zur Verwendung als Arzneimittel.

16. Magnetpartikel-haltiges Aerosol nach einem der Ansprüche 1 bis 12 zur Prophylaxe und/ oder Therapie von Atemwegs- und/oder Lungenerkrankungen, insbesondere von entzündlichen und/oder obstruktiven Atemwegs- und/oder Lungenerkrankungen ausgewählt aus Melanomen oder malignen Melanomen der Atemwege, insbesondere der Lunge, Lungenkrebs, Lungentumoren, Lungenkarzinomen, kleinzelligen Lungenkarzinomen, Rachenkrebs, Bronchialkarzinomen, Kehlkopfkrebs, Kopf-Hals-Tumoren, Zungenkrebs, Sarkomen und Blastomen im Bereich oder Nähe der Atemwege, insbesondere der Lunge, sowie Asthma, COPD ("chronic obstructive pulmonary disease"; chronisch obstruktive Lungenerkrankung), Lungenemphysem, chronischer Bronchitis, Lungenentzündung sowie Erbkrankheiten, bspw. Mukoviszidose, humanem Surfactant Protein B-Mangel und $\alpha_1$-Antitrypsinmangel, sowie zur Therapie nach einer Lungentransplantation sowie zur pulmonalen Vakzininierung und zur antiinfektien Therapie der Lunge.

17. Verwendung eines Magnetpartikel-haltigen Aerosols nach einem der Ansprüche 1 bis 12 zur Herstellung eines Arzneimittels zur Prophylaxe und/oder Therapie von Atemwegs- und/oder Lungenerkrankungen, insbesondere von entzündlichen und/oder obstruktiven Atemwegs- und/oder Lungenerkrankungen, ausgewählt aus Melanomen oder malignen Melanomen der Atemwege, insbesondere der Lunge, Lungenkrebs, Lungentumoren, Lungenkarzinomen, kleinzelligen Lungenkarzinomen, Rachenkrebs, Bronchialkarzinomen, Kehlkopfkrebs, Kopf-Hals-Tumoren, Zungenkrebs, Sarkomen und Blastomen im Bereich oder Nähe der Atemwege, insbesondere der Lunge, sowie Asthma und COPD, Lungenemphysem, chronischer Bronchitis, Lungenentzündung sowie Erbkrankheiten, bspw. Mukoviszidose, humanem Surfactant Protein B-Mangel und $\alpha_1$-Antitrypsinmangel, sowie zur Therapie nach einer Lungentransplantation sowie zur pulmonalen Vakzininierung und zur antiinfektien Therapie der Lunge.

18. Magnetpartikel-haltiges Aerosol zur Verwendung nach Anspruch 15 oder 16 oder Verwendung nach Anspruch 17, wobei das Magnetpartikel-haltige Aerosol durch ein Magnetfeld auf der Oberfläche des zu therapierenden Atemwegs- und/oder Lungenbereichs deponiert wird.

19. Magnetpartikel-haltiges Aerosol zur Verwendung oder Verwendung nach Anspruch 18, wobei das Magnetfeld eine Feldstärke von mindestens 100 mT (Millitesla), mindestens 200 mT, mindestens 500 mT, oder mindestens 1 T (Tesla) aufweist.

20. Magnetpartikel-haltiges Aerosol zur Verwendung oder Verwendung nach Anspruch 18, wobei das Magnetfeld einen Magnetfeldgradienten von größer als 1 T/m oder größer als 10 T/m aufweist.

21. Magnetpartikel-haltiges Aerosol zur Verwendung oder Verwendung nach einem der Ansprüche 18 bis 20, wobei das Magnetfeld ein pulsierendes, ein oszillierendes oder ein pulsierendes-oszillierendes Magnetfeld ist.

22. Magnetpartikel-haltiges Aerosol zur Verwendung oder Verwendung nach einem der Ansprüche 18 bis 20, wobei das Magnetfeld dynamisch an die Atmung des Patienten angepasst ist und lediglich während der Ruhepausen zwischen Ein- und Ausatmen bzw. Aus- und Einatmen aktiv ist.

23. Vorrichtung zur Verneblung und/oder Verabreichung eines Magnetpartikel-haltigen Aerosols, enthaltend einen Vernebler, wobei der Vernebler mit Magnetpartikel-haltigen Aerosol nach einem der Ansprüche 1 bis 12 befüllt ist.

**Claims**

1. Aerosol containing magnetic particles, wherein the aerosol contains magnetic particles having a diameter of at least 5 nm and at most 800 nm and at least one pharmaceutical active agent.

2. Aerosol containing magnetic particles according to claim 1, wherein the magnetic particles have a diameter of at least 50 nm and at most 750 nm, preferably of at least 100 nm and at most 700 nm, more preferably of at least 150 nm and at most 600 nm, still more preferably of at least 200 nm and at most 500 nm, particularly preferably of at least 250 nm and at most 450 nm, most preferably of at least 300 nm and at most 400 nm.

**3.** Aerosol containing magnetic particles according to claim 1 or 2, wherein the magnetic particles and the pharmaceutical active agent are contained in a solvent.

**4.** Aerosol containing magnetic particles according to claim 3, wherein the solvent is an inorganic or organic solvent.

**5.** Aerosol containing magnetic particles according to claim 3 or 4, wherein the solvent is selected from the group consisting of ethanol, water and glycerol and mixtures thereof.

**6.** Aerosol containing magnetic particles according to one of claims 1 to 5, wherein the pharmaceutical active agent is coupled to the magnetic particles.

**7.** Aerosol containing magnetic particles according to one of claims 1 to 5, wherein the pharmaceutical active agent is not coupled to the magnetic particles.

**8.** Aerosol containing magnetic particles according to one of claims 1 to 7, wherein the pharmaceutical active agent is selected from the group consisting of nucleic acids, peptides, proteins, cytostatics, broncholytics, antibiotics, antidiabetics and immunomodulators.

**9.** Aerosol containing magnetic particles according to one of claims 1 to 8, wherein the pharmaceutical active agent is contained in a vector, liposome, hollow colloid or nanoparticle.

**10.** Aerosol containing magnetic particles according to one of claims 1 to 9, wherein the magnetic particles consist of metals and/or oxides and/or hydroxides thereof or contain these.

**11.** Aerosol containing magnetic particles according to one of claims 1 to 10, wherein the magnetic particles consist of metals, or contain these, selected from the group consisting of iron, cobalt or nickel, magnetic iron oxides or hydroxides, such as $Fe_3O_4$, gamma-$Fe_2O_3$, double oxides or hydroxides of di- or trivalent iron ions with other di- or trivalent metal ions, such as $Co^{2+}$, $Mn^{2+}$, $Cu^{2+}$, $Ni^{2+}$, $Cr^{3+}$, $Gd^{3+}$, $Dy^{3+}$ or $Sm^{3+}$, and any mixtures thereof.

**12.** Aerosol containing magnetic particles according to one of claims 1 to 11, wherein the magnetic particles consist of paramagnetic or superparamagnetic material or contain this.

**13.** Pharmaceutical composition comprising an aerosol containing magnetic particles according to one of claims 1 to 12, and optionally suitable auxiliary substances and/or additives.

**14.** Pharmaceutical composition according to claim 13, additionally comprising at least one (further) solvent, at least one complexing agent and/or at least one pharmaceutically acceptable acid.

**15.** Aerosol containing magnetic particles according to one of claims 1 to 12 for use as a medicament.

**16.** Aerosol containing magnetic particles according to one of claims 1 to 12 for use for prophylaxis and/or therapy of diseases of the respiratory tract and/or lungs, in particular inflammatory and/or obstructive diseases of the respiratory tract and/or lungs selected from melanomas or malignant melanomas of the respiratory tract, in particular the lung, lung cancer, lung tumours, lung carcinomas, small cell lung carcinomas, throat cancer, bronchial carcinomas, larynx cancer, head/neck tumours, tongue cancer, sarcomas and blastomas in the region or vicinity of the respiratory tract, in particular the lung, as well as asthma, COPD (chronic obstructive pulmonary disease), lung emphysema, chronic bronchitis, pneumonia and hereditary diseases, for example mucoviscidosis, human surfactant protein B deficiency and $\alpha_1$-antitrypsin deficiency, and for therapy after a lung transplant and for pulmonary vaccination and for anti-infective therapy of the lung.

**17.** Use of an aerosol containing magnetic particles according to one of claims 1 to 12 for the preparation of a medicament for prophylaxis and/or therapy of diseases of the respiratory tract and/or lungs, in particular inflammatory and/or obstructive diseases of the respiratory tract and/or lungs selected from melanomas or malignant melanomas of the respiratory tract, in particular the lung, lung cancer, lung tumours, lung carcinomas, small cell lung carcinomas, throat cancer, bronchial carcinomas, larynx cancer, head/neck tumours, tongue cancer, sarcomas and blastomas in the region or vicinity of the respiratory tract, in particular the lung, as well as asthma and COPD, lung emphysema, chronic bronchitis, pneumonia and hereditary diseases, for example mucoviscidosis, human surfactant protein B deficiency and $\alpha_1$-antitrypsin deficiency, and for therapy after a lung transplant and for pulmonary vaccination and for anti-infective therapy of the lung.

18. Aerosol containing magnetic particles for use according to one of claims 15 or 16 or use according to claim 17, wherein the aerosol containing magnetic particles is deposited by a magnetic field onto the surface of the region of the respiratory tract and/or lung to be treated.

19. Aerosol containing magnetic particles for use or use according to claim 18, wherein the magnetic field has a field strength of at least 100 mT (millitesla), at least 200 mT, at least 500 mT or at least 1 T (tesla).

20. Aerosol containing magnetic particles for use or use according to claim 18, wherein the magnetic field has a magnetic field gradient of greater than 1 T/m or greater than 10 T/m.

21. Aerosol containing magnetic particles for use or use according to one of claims 18 to 20, wherein the magnetic field is a pulsating, an oscillating or a pulsating-oscillating magnetic field.

22. Aerosol containing magnetic particles for use or use according to one of claims 18 to 20, wherein the magnetic field is matched dynamically to the breathing of the patient and is active only during the resting pauses between in- and exhalation or ex- and inhalation.

23. A device for atomizing and/or administering an aerosol containing magnetic particles containing an atomizer, wherein the atomizer is filled with an aerosol containing magnetic particles according to one of claims 1 to 12.

**Revendications**

1. Aérosol contenant des particules magnétiques, l'aérosol contenant des particules magnétiques ayant un diamètre d'au moins 5 nm et d'au plus 800 nm ainsi qu'au moins un principe actif pharmaceutique.

2. Aérosol contenant des particules magnétiques selon la revendication 1, les particules magnétiques présentant un diamètre d'au moins 50 nm et d'au plus 750 nm, de préférence d'au moins 100 nm et d'au plus 700 nm, de manière davantage préférée d'au moins 150 nm et d'au plus 600 nm, de manière encore davantage préférée d'au moins 200 nm et d'au plus 500 nm, de manière particulièrement préférée d'au moins 250 nm et d'au plus 450 nm et de manière préférée entre toutes d'au moins 300 nm et d'au plus 400 nm.

3. Aérosol contenant des particules magnétiques selon la revendication 1 ou 2, les particules magnétiques et le principe actif pharmaceutique étant contenus dans un solvant.

4. Aérosol contenant des particules magnétiques selon la revendication 3, le solvant étant un solvant organique ou inorganique.

5. Aérosol contenant des particules magnétiques selon la revendication 3 ou 4, le solvant étant choisi dans le groupe constitué de l'éthanol, de l'eau et de la glycérine ainsi que des mélanges de ceux-ci.

6. Aérosol contenant des particules magnétiques selon l'une des revendications 1 à 5, le principe actif pharmaceutique étant fixé aux particules magnétiques.

7. Aérosol contenant des particules magnétiques selon l'une des revendications 1 à 5, le principe actif pharmaceutique n'étant pas fixé aux particules magnétiques.

8. Aérosol contenant des particules magnétiques selon l'une des revendications 1 à 7, le principe actif pharmaceutique étant choisi dans le groupe constitué des acides nucléiques, des peptides, des protéines, des cytostatiques, des bronchiolytiques, des antibiotiques, des antidiabétiques et des immunomodulateurs.

9. Aérosol contenant des particules magnétiques selon l'une des revendications 1 à 8, le principe actif pharmaceutique étant contenu dans un vecteur, un liposome, un colloïde creux ou une nanoparticule.

10. Aérosol contenant des particules magnétiques selon l'une des revendications 1 à 9, les particules magnétiques étant constituées de métaux et/ou de leurs oxydes et/ou hydroxydes ou bien contenant ceux-ci.

**11.** Aérosol contenant des particules magnétiques selon l'une des revendications 1 à 10, les particules métalliques étant constituées de métaux ou bien contenant ceux-ci, choisis dans le groupe constitué du fer, du cobalt ou du nickel, des oxydes ou hydroxydes de fer magnétiques comme $Fe_3O_4$, $\gamma$-$Fe_2O_3$, les oxydes ou hydroxydes doubles constitués d'ions fer divalents ou trivalents avec d'autres ions métal divalents ou trivalents comme $Co^{2+}$, $Mn^{2+}$, $Cu^{2+}$, $Ni^{2+}$, $Cr^{3+}$, $Gd^{3+}$, $Dy^{3+}$ ou $Sm^{3+}$ ainsi que des mélanges quelconques de ceux-ci.

**12.** Aérosol contenant des particules magnétiques selon l'une des revendications 1 à 11, les particules magnétiques étant constituées de matière paramagnétique ou superparamagnétique ou bien contenant celle-ci.

**13.** Composition pharmaceutique renfermant un aérosol contenant des particules magnétique selon l'une des revendications 1 à 12 ainsi que des auxiliaires et/ou des additifs adaptés le cas échéant.

**14.** Composition pharmaceutique selon la revendication 13, contenant de plus au moins un (autre) solvant, au moins un agent complexant et/ou au moins un acide pharmaceutiquement acceptable.

**15.** Aérosol contenant des particules magnétiques selon l'une des revendications 1 à 12 à utiliser comme médicament.

**16.** Aérosol contenant des particules magnétiques selon l'une des revendications 1 à 12 destiné à la prophylaxie et/ou au traitement des maladies respiratoires et/ou pulmonaires, notamment des maladies respiratoires et/ou pulmonaires inflammatoires et/ou obstructives choisies parmi les mélanomes ou les mélanomes malins des voies respiratoires, notamment des poumons, le cancer du poumon, les tumeurs pulmonaires, les carcinomes pulmonaires, les carcinomes pulmonaires à petites cellules, le cancer du pharynx, les carcinomes bronchiques, le cancer du larynx, les tumeurs de la tête et du cou, le cancer de la langue, les sarcomes et les blastomes des voies respiratoires ou à proximité des voies respiratoires, notamment du poumon, ainsi que l'asthme, la BPCO (broncho-pneumopathie chronique obstructive), l'emphysème pulmonaire, la bronchite chronique, l'inflammation des poumons de même que les maladies génétiques comme la mucoviscidose, le déficit en protéine B du surfactant pulmonaire et le déficit en $\alpha_1$-antitrypsine et également destiné au traitement des transplantés pulmonaires ainsi qu'à la vaccination contre les maladies pulmonaires et au traitement anti-infectieux des poumons.

**17.** Utilisation d'un aérosol contenant des particules magnétiques selon l'une des revendications 1 à 12 pour la fabrication d'un médicament destiné à la prophylaxie et/ou au traitement des maladies respiratoires et/ou pulmonaires, notamment des maladies respiratoires et/ou pulmonaires inflammatoires et/ou obstructives choisies parmi les mélanomes ou les mélanomes malins des voies respiratoires, notamment des poumons, le cancer du poumon, les tumeurs pulmonaires, les carcinomes pulmonaires, les carcinomes pulmonaires à petites cellules, le cancer du pharynx, les carcinomes bronchiques, le cancer du larynx, les tumeurs de la tête et du cou, le cancer de la langue, les sarcomes et les blastomes des voies respiratoires ou à proximité des voies respiratoires, notamment du poumon, ainsi que l'asthme, la BPCO, l'emphysème pulmonaire, la bronchite chronique, l'inflammation des poumons de même que les maladies génétiques comme la mucoviscidose, le déficit en protéine B du surfactant pulmonaire et le déficit en $\alpha_1$-antitrypsine et également destiné au traitement des transplantés pulmonaires ainsi qu'à la vaccination contre les maladies pulmonaires et au traitement anti-infectieux des poumons.

**18.** Aérosol contenant des particules magnétiques à utiliser selon la revendication 15 ou 16 ou utilisation selon la revendication 17, l'aérosol contenant des particules magnétiques étant déposé par l'intermédiaire d'un champ magnétique à la surface de la zone respiratoire et/ou pulmonaire à traiter.

**19.** Aérosol contenant des particules magnétiques à utiliser ou utilisation selon la revendication 18, le champ magnétique présentant une intensité d'au moins 100 mT (millitesla), d'au moins 200 mT, d'au moins 500 mT ou d'au moins 1 T (tesla).

**20.** Aérosol contenant des particules magnétiques à utiliser ou utilisation selon la revendication 18, le champ magnétique présentant des gradients de champ magnétique de plus de 1 T/m ou de plus de 10 T/m.

**21.** Aérosol contenant des particules magnétiques à utiliser ou utilisation selon l'une des revendications 18 à 20, le champ magnétique étant un champ magnétique pulsé, oscillant ou oscillant et pulsé.

**22.** Aérosol contenant des particules magnétiques à utiliser ou utilisation selon l'une des revendications 18 à 20, le champ magnétique s'adaptant de manière dynamique à la respiration du patient et étant uniquement actif durant les pauses entre chaque inspiration et expiration ou chaque expiration et inspiration.

23. Dispositif pour nébuliser et/ou administrer un aérosol contenant des particules magnétiques comprenant un nébuliseur, le nébuliseur étant rempli d'aérosol contenant des particules magnétiques selon l'une des revendications 1 à 12.

**Impaktion**
(Trägheit), >3μm

**Diffusion**
(Brownsche Molekularbewegung),
<2μm

**Sedimentation**
(Schwerkraft), 0,5-4μm

Schematische Darstellung der Depostionsmechanismen
in Abhängigkeit des Partikeldurchmessers

# Figur 1

Partikeldeposition in der Lunge

**Figur 2**

Verteilung der eingebrachten Lösung von
2,5 ml nach Inhalation von mindestens 25 Minuten

Ergebnisse

Restmenge
im Vernebler
1431 +/-153 mg

Exhalation
530 +/-86 mg

21,3%

59,7

extrabronchial
135 +/-47 mg

5,6%

13,4%

intrabronchial
317 +/-167 mg

PARI IS-2 Vernebler; n=10

**Partikeldeposition in der Lunge**

# Figur 3

Glottis

Lunge dorsal

**Depositionsmuster herkömmlich applizierter Aerosoli in der Lunge**

## Figur 4

**Barrieren in der Lunge**

**Figur 5**

**A**

**B**

Darstellung von Zellen aus der Mukusschicht der Lunge mit (A) und ohne (B) Magnetpartikeldeposition

## Figur 6

**Konzept der Magnetfeld-gesteuerten Aerosolapplikation**

# Figur 7

**Schematischer Aufbau des Tierversuches zur Magnetfeld-gesteuerten Aerosolapplikation**

# Figur 8

**Aufbau des Tierversuches zur Magnetfeld-gesteuerten Aerosolapplikation**

**Figur 9**

**rechte Lunge**  **linke Lunge**  **Kontrolle**

Histologische Auswertung des Tierversuches zur Magnetfeld-gesteuerten Aerosolapplikation

**Figur 10**

Quantitative Auswertung des Tierversuches zur Magnetfeld-gesteuerten
Aerosolapplikation; Thorax eröffnet, Elektromagnet auf rechter Lunge platziert.

## Figur 11

**Quantitative Auswertung des Tierversuches zur Magnetfeld-gesteuerten Aerosolapplikation; Thorax geschlossen, Elektromagnet auf rechter Lunge platziert.**

**Figur 12**

Deponierte Menge pCMVLuc in 80ng genomischer DNA der Lunge

**Figur 13**

$\varnothing= 7mm$

$v_{aer}=4,76$ m/s

**Schematischer Versuchsaufbau zur Untersuchung der Magnetfeld-induzierten Aerosolablenkung**

**Figur 14**

NdFeB (20 Ø x 5 mm)

AlNiCo (20 Ø x 120 mm)

Ø= 7mm

$v_{aer}$=4,76 m/s

t=10 min

c=55 mg/ml (fluidMAG 14/N)

12    13    14    15    16    17    18

Magnetfeld-induzierte Aerosolablenkung superparamagnetische Eisenoxidpartikel-haltiger Aerosole.

**Figur 15**

NdFeB (20 Ø x 5 mm)

AlNiCo (20 Ø x 120 mm)

560 mT

410 mT

Ø= 7mm

$v_{aer}$=4,76 m/s

t=10 min

c=55 mg/ml (fluidMAG 14/N)

**Magnetfeld-induzierte Aerosolablenkung superparamagnetische Eisenoxidpartikel-haltiger Aerosole.**

**Figur 16**

**Einfluss des Parameters der Feldstärke (T) auf die Aerosolablenkung (cm)**

**Figur 17**

**Einfluss des Parameters des Magnetpartikeldurchmessers [d (nm)]auf die Aerosolablenkung [A (cm$^2$)]**

# Figur 18

Einfluss des Parameters der Eisenkonzentration [c (mg/ml)] auf die Aerosolablenkung
[A (cm$^2$)]

**Figur 19**

| Feldstärke (mT) | relative Depositionsrate (%) | Abnahme verglichen mit einer Konzentration von 25 mg/ml |
|---|---|---|
| 1000 | 100 | - |
| 870 | 76 | 1.3-fach |
| 730 | 68 | 1.5-fach |

Ergebnisse der Figur 17 in tabellarischer Form

| diameter magenetic particle (nm) | relative deposition rate (%) | Abnahme verglichen mit einem Durchmesser von 50 nm |
|---|---|---|
| 50 | 100 | - |
| 100 | 80 | 1.25-fach |
| 400 | 81 | 1.23-fach |
| 1000 | 0 | > 100-fach |
| 2000 | 0 | > 100-fach |

Ergebnisse der Figur 18 in tabellarischer Form

| ion concentration (mg/ml) | relative deposition rate (%) | Abnahme verglichen mit einer Konzentration von 25 mg/ml |
|---|---|---|
| 50.0 | 88 | 1.14-fach |
| 25.0 | 100 | - |
| 12.5 | 5 | 19-fach |
| 6.3 | 0 | > 100-fach |
| 3.1 | 0 | > 100-fach |
| 1.6 | 0 | > 100-fach |
| 0.8 | 0 | > 100-fach |

Ergebnisse der Figur 19 in tabellarischer Form

# Figur 20

**Figur 21**

**Figur 22**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 02000870 A
- DE 19624426
- US 4554088 A
- US 4554089 A
- US 4208294 A
- US 4101435 A
- WO 9114468 A
- WO 9712687 A
- WO 9407607 A
- WO 9916530 A

**In der Beschreibung aufgeführte Nicht-Patentliteratur**

- **Köhler, D. ; Fischer, F.** Theorie und Praxis der Inhalationstherapie. Arcis Verlag, 2000
- **Ernst, N. et al.** Interaction of liposomal and polycationic transfection complexes with pulmonary surfactant. *J Gene Med,* 1999, vol. 1, 331-40
- **Rosenecker, J. et al.** Interaction of bronchoalveolar lavage fluid with polyplexes and lipoplexes: analysing the role of proteins and glycoproteins. *J Gene Med,* 2003, vol. 5, 49-60
- **Rudolph, C. et al.** Nonviral gene delivery to the lung with copolymer-protected and transferrin-modified polyethylenimine. *Biochim Biophys Acta,* 2002, vol. 1573, 75-83
- **Ally et al.** *Journal of Magnetism and Magnetic Materials,* 2005, vol. 293, 442-449
- **Schwertmann U. ; Cornell R. M.** Iron Oxides in the Laboratory. VCH Weinheim, 1991
- **Cryan S-A.** Carrier-based Strategies for Targeting Protein and Peptide Drugs to the Lungs. *AAPS Journal.,* 2005, vol. 07 (01), E20-E41
- **Sambrook et al.** Molecular Cloning A Laboratory Manual. Cold Spring Harbor Laboratory, 1989
- **Koshkina, N. V. et al.** Paclitaxel liposome aerosol treatment induces inhibition of pulmonary metastases in murine renal carcinoma model. *Clinical Cancer Research,* 2001, vol. 7, 3258-3262
- **von Greg T. Hermanson.** Bioconjugate Techniques. Academic Press, 01. Januar 1996
- **Druckschrift.** Theorie und Praxis der Inhalationstherapie. Arcis Verlag, 2000, 31-70
- Theorie und Praxis der Inhalationstherapie. Arcis Verlag, 2000, 31-70
- **Sanders, N. N. ; De Smedt, S. C. ; Demeester, J.** The physical properties of biogels and their permeability for macromolecular drugs and colloidal drug carriers [Review]. *Journal of Pharmaceutical Sciences,* 2000, vol. 89, 835-849
- **Lange J et al.** Magnetorelaxometry, a new binding specific detection method based on magnetic nanoparticles. *JOURNAL OF MAGNETISM AND MAGNETIC MATERIALS,* November 2002, vol. 252 (1-3), 381-383
- **Rudolph C ; Ortiz A ; Schillinger U ; jauernig J ; Plank C ; Rosenecker J.** Methodological optimization of polyethylenimine (PEI)-based gene delivery to the lungs of mice via aerosol application. *J Gene Med.,* Januar 2005, vol. 7 (1), 59-66
- **Rudolph C ; Schillinger U ; Ortiz A ; Plank C ; Golas MM ; Sander B ; Stark H ; Rosenecker J.** Aerosolized nanogram quantities of plasmid DNA mediate highly efficient gene delivery to mouse airway epithelium. *Mol Ther.,* September 2005, vol. 12 (3), 493-501